# EUROPEAN PATENT APPLICATION

(11) **EP 3 536 784 A1**
(43) Date of publication of application: **11.09.2019**
(21) Application number: 18159931.7
(22) Date of filing: 05.03.2018
(51) Int. Cl.: C12N 15/09

(54) **HOST CELL ENGINEERED FOR IMPROVED METABOLITE PRODUCTION**

(71) Applicant: ACIB GmbH, 8010 Graz (AT); Universität für Bodenkultur Wien, 1180 Wien (AT)
(72) Inventor: STEIGER, Matthias, 1120 Wien (AT); SAUER, Michael, 1040 Vienna (AT); MATTANOVICH, Diethard, 1180 Vienna (AT)
(74) Representative: Redl, Gerda

(57) **Abstract**

A host cell comprising a modification for modulated expression of a citrate export gene A (cexA), wherein the *cexA* gene encodes a protein which has at least 70% sequence identity to SEQ ID NO:1 (CexA), and a method of producing a fermentation product other than citric acid by culturing the host cell under conditions that permit the host cell to produce the fermentation product, and isolating the produced fermentation product.

## Description

### TECHNICAL FIELD

The invention relates to a host cell which is engineered for improved cell metabolite production, in particular by modulating, *i.e.* increasing or reducing the expression of a citric acid transport gene

### BACKGROUND OF THE INVENTION

Since more than 50 years, *Aspergillus niger* strains are used as industrial microorganism to produce organic acids and as most important product citric acid. Citric acid is readily produced by *A. niger* strains selected by classic strain breeding and screening techniques involving *e.g.*, UV mutagenesis (Karaffa and Kubicek, Appl. Microbiol. Biotechnol. 2003, 61:189-96).

Almost all enzymatic steps in the biosynthesis pathway were characterized in the past. Since the genome of *A. niger* was released, almost all genes responsible for the biosynthesis of citric acid were identified (Andersen et al., Mol. Syst. Biol. 2008, 4:178). Citric acid is produced inside the fungal host and secreted to the medium, where it accumulates to concentrations exceeding 200 g/L. Targeted strain engineering to enhance citrate is possible in *A. niger* (de Jongh and Nielsen, Metab. Eng. 2008. 10:87-96), but requires the genome information (Andersen et al., Genome Res. 2011, 21, 885-97; Andersen et al. 2008; Pel et al., Nat. Biotechnol. 2007, 25:221-31). However, the annotation of the genome is not complete. Therefore, the information about certain metabolic pathways is still fragmented. Especially the information on the genetic identity of the *A. niger* citrate export system is missing.

The uptake as well as the export of citric acid by *A. niger* occur by active, delta pH-driven, H(+)-symport dependent systems. Citrate export was demonstrated in a mycelium cultivated under manganese-deficient growth conditions. (Netik et al., Biochim. Biophys. Acta 1997, 1326:287-94).

Up to now, the genetic identity of the citric acid transport system is not known for *A. niger.* However, in the past the export system was already described to be an important part of the biosynthesis pathway based on theoretical calculations (Karaffa and Kubicek, 2003; Torres, Biotechnol. Bioeng. 1994, 44:104-11).

A list of 46 putative citrate transporter candidates was recently published (Yin et al., Sci. Rep. 2017, 7:1-16).

Steiger et al. (28th Fungal Genetics Conference, March 17-22, 2015, Pacific Grove, USA) describe transport proteins for itaconic acid production in *A. niger.*

Sarkari et al. (Bioresour Technol 2017, 245(Pt B):1327-1333) describe genetic tools for metabolic engineering of *A. niger* to introduce heterologous expression cassettes at a defined genetic locus.

There is a need to provide genetic information about the transport processes in *A. niger* to perform targeted strain engineering on the citrate export system.

### SUMMARY OF THE INVENTION

It is the object to engineer an improved host cell which has improved protein or metabolite production capabilities targeting citrate export in said cell, either to promote citrate transport, or to reduce citrate transport.

The object is solved by the subject of the claims and further described herein.

According to the invention, there is provided a host cell comprising a modification for modulated expression of a citrate export gene A (*cexA*), wherein the *cexA* gene encodes a protein which has at least 70% sequence identity to SEQ ID NO:1 (CexA).

Specifically, CexA has at least any one of 70%, 80%, 90%, or 95% sequence identity to SEQ ID NO:1.

Specifically, CexA is functional as an export protein, exporting organic acids, in particular citric acid into a cell culture medium.

According to a specific aspect, CexA is endogenous or heterologous to the host cell.

Specifically, CexA is naturally occurring in any prokaryotic or eukaryotic cell, in particular in a microbial cell, such as *e.g*., a filamentous fungus, yeast, or mammalian cells, or an artificial protein that is homologous to a naturally-occurring sequence, in particular an artificial protein obtainable by mutagenesis of a naturally occurring CexA, which comprises one or more mutations in the amino acid sequence, and has any one of 70%, 80%, 90%, or 95% sequence identity to SEQ ID NO:1.

Specifically, CexA is endogenous to the host cell, in particular naturally occurring in the wild-type host cell.

Specifically, CexA comprises or consists of SEQ ID NO:1. Specifically, CexA is of *A. niger* origin, or an artificial protein obtainable by mutagenesis of such naturally occurring CexA of *A. niger* origin, which comprises one or more mutations in the amino acid sequence, and has any one of 70%, 80%, 90%, or 95% sequence identity to SEQ ID NO:1.

Specifically, CexA is a homolog or an ortholog of the CexA protein which is naturally occurring in a species other than *A. niger* and which comprises or consists of SEQ ID NO:1, or an artificial protein obtainable by mutagenesis of such naturally occurring CexA, which comprises one or more mutations in the amino acid sequence, and has any one of 70%, 80%, 90%, or 95% sequence identity to SEQ ID NO:1.

The host cell is specifically provided as a production host cell or the respective host cell line, which is the result of genetic engineering, and thus man-made and artificial. Animals and human beings are particularly excluded from the claims.

Specifically, the host cell is a microbial cell, *e.g*., a eukaryotic microbial cell, such as a filamentous fungal cell, preferably *Aspergillus, Trichoderma, Fusarium, Penicillium*, or yeast, preferably *Pichia, Yarrowia, Hansenula, Komagataella,* or *Saccharomyces.*

Specifically, the host cell is of *Aspergillus niger,* preferably any of the *A. niger* strain American Type Culture Collection (ATCC) 1015, ATCC 9029, ATCC 9142, ATCC 10577, ATCC 11414, ATCC 12846, ATCC 16404, ATCC 26550, ATCC 201122, ARS Culture Collection National Center for Agricultural Utilization Research (NRRL) NRRL 2270, Microbial Culture Collection of the National Institute of chemistry (MZKI), Ljubljana, Slovenia MZKI A-60, MZKI A-138, CBS 513.88 or a progeny, descendant or mutant strain of any of the foregoing.

Specifically the host cell is a cell of a genus selected from the group consisting of *Aspergillus, Trichoderma, Penicillium, Pichia, Hansenula, Komagataella, Saccharomyces, Kluyveromyces, Candida, Ogataea, Yarrowia,* and *Geotrichum, specifically, Aspergillus oryzae, Aspergillus aculeatus, Aspergillus clavatus, Aspergillus wentii, Aspergillus luchuensis, Aspergillus terreus, Aspergillus flavus, Aspergillus neoniger, Aspergillus tubingensis, Aspergillus awamori, Aspergillus carbonarius, A. brasiliensis, Penicillium chryosogenum, Trichoderma reesei, Trichoderma atroviride, Saccharomyces cerevisiae, Pichia pastoris, Ogataea minuta or Hansenula polymorpha, Yarrowia lipolytica, Aspergillus awamori* or *Trichoderma reesei.* Preferably, the host cell is a methylotrophic yeast, preferably *Pichia pastoris.* Herein *Pichia pastoris* is used synonymously for all, *Komagataella pastoris, Komagataella phaffii* and *Komagataella pseudopastoris.*

Specifically, the host cell is a *Pichia pastoris* strain selected from the group consisting of CBS 704, CBS 2612, CBS 7435, CBS 9173-9189, DSMZ 70877, X-33, GS115, KM71, KM71 H and SMD1168.

Sources: CBS 704 (=NRRL Y-1603 = DSMZ 70382), CBS 2612 (=NRRL Y-7556), CBS 7435 (=NRRL Y-11430), CBS 9173-9189 (CBS strains: CBS-KNAW Fungal Biodiversity Centre, Centraalbureau voor Schimmelculturen, Utrecht, The Netherlands), and DSMZ 70877 (German Collection of Microorganisms and Cell Cultures); strains from Invitrogen, such as X-33, GS115, KM71, KM71H and SMD1168. Examples of *S. cerevisiae* strains include W303, CEN.PK and the BY-series (EUROSCARF collection).

Specifically, the host cell is a mammalian cell, such as a human, rodent or bovine cell. Examples of specific mammalian cells suitable as host cells described herein are mouse myeloma (NSO)-cell lines, Chinese hamster ovary (CHO)-cell lines, HT1080, H9, HepG2, MCF7, MDBK Jurkat, MDCK, NIH3T3, PC12, BHK (baby hamster kidney cell), VERO, SP2/0, YB2/0, Y0, C127, L cell, COS, e.g., COS1 and COS7, QC1-3, HEK-293, VERO, PER.C6, HeLA, EBI, EB2, EB3, oncolytic or hybridoma-cell lines. Preferably the mammalian cells are CHO-cell lines.

Specifically, the host cell is a bacterial cell, in particular a Gram-positive bacterial cell.

Specifically, the host cell is selected from the genus *Escherichia,* such as *E. coli, or* Bacillus, such as *Bacillus subtilis, Actobacillus, Rhodococcus, or Pseudomonas.*

All of the strains described herein have been successfully used to produce transformants and to express heterologous genes.

Any of the cells or strains described herein can be advantageously used to produce mutants which are capable of both, a modulated citric acid export, including e.g., secretion or transport, and production of a fermentation product by such cells and strains, respectively. Specifically, fermentation products are produced into the cell culture medium, and obtained from the cell culture medium.

According to a specific aspect, the *cexA* gene is endogenous or heterologous to the host cell, preferably wherein the *cexA* gene has at least 70% sequence identity to SEQ ID NO:2.

Specifically, the *cexA* gene has at least any one of 70%, 80%, 90%, or 95% sequence identity to SEQ ID NO:2.

Specifically, the *cexA* gene is naturally occurring in any prokaryotic or eukaryotic cell, in particular in a microbial cell, such as *e.g.,* a filamentous fungus, yeast, or mammalian cells, or an artificial protein that is homologous to a naturally-occurring sequence, in particular an artificial protein obtainable by mutagenesis of a naturally occurring CexA, which comprises one or more mutations in the amino acid sequence, and has any one of 70%, 80%, 90%, or 95% sequence identity to SEQ ID NO:1.

Specifically, the *cexA* gene is endogenous to the host cell, in particular naturally occurring in the wild-type host cell.

Specifically, the *cexA* gene comprises or consists of SEQ ID NO:2. Specifically, the *cexA* gene is of *A. niger* origin, such as of wild-type *A. niger,* or an artificial polynucleotide or gene obtainable by mutagenesis of such naturally occurring *cexA* gene of *A. niger* origin, which comprises one or more mutations in the nucleic acid sequence, and has any one of 70%, 80%, 90%, or 95% sequence identity to SEQ ID NO:2.

Specifically, the *cexA* gene is a homolog or an ortholog of the *cexA* gene which is naturally occurring in a species other than *A. niger* and which comprises or consists of SEQ ID NO:2, or an artificial protein obtainable by mutagenesis of such naturally occurring *cexA* gene, which comprises one or more mutations in the nucleic acid sequence, and has any one of 70%, 80%, 90%, or 95% sequence identity to SEQ ID NO:2.

According to a specific aspect, the modification is a genetic modification, specifically wherein the genetic modification results in a genomic mutation. Specifically, the genetic modification results in at least one heterologous polynucleotide within the genome of the host cell.

Specifically, the heterologous polynucleotide comprises a DNA or RNA sequence, preferably comprising a non-coding or coding sequence within a chromosome or a plasmid.

Specifically, the heterologous polynucleotide is within an expression cassette expressing the *cexA* gene, preferably wherein the heterologous polynucleotide comprises a promoter and/or gene sequence.

Specifically, the cell has been transformed with said expression cassette.

Specifically, the cell has been transformed with a vector comprising said expression cassette.

According to a specific embodiment, the genetic modification includes transformation of the host cell to incorporate an expression cassette, employing one or more heterologous nucleic acid sequences. Specifically, the expression cassette comprises a *cexA* gene as described herein.

Specifically, the expression cassette comprises one or more regulatory nucleic acid sequences operably linked to a nucleotide sequence encoding the CexA to be produced, in particular wherein said one or more operably linked sequences are not naturally associated with the coding sequence.

Specifically, at least one of the expression control sequences is a promoter operably linked to any of the heterologous nucleic acid molecules, which promoter is not naturally-occurring in the host cell and/or not natively associated with said operably linked heterologous nucleic acid molecule. Specifically, expression of each of the heterologous nucleic acid molecules is controlled by a promoter in operable linkage to the respective heterologous nucleic acid molecule, wherein said promoter is not naturally-occurring in the host cell and/or not natively associated with said operably linked heterologous nucleic acid molecule.

Specifically, the expression cassette comprises a promoter operably linked to the gene encoding the CexA to be produced.

Specifically, the expression cassette comprises a constitutive or inducible or repressible promoter.

Specific examples of a constitutive promoter include promoters suitable for expressing sequences in *A. niger,* such as *A. niger* promoters *e.g*., pglaA (Fowler et al., 1990, Curr Genet. 18(6):537-45). pgpdA, (Punt et al. 1991, J Biotechnol.; 17(1):19-33) pmbfA, pcoxA, psrpB, ptvdA, pmdhA, pmanB, (Blumhoff et al., 2013, Appl Microbiol Biotechnol.;97(1):259-67), pGAP and functional variants thereof,or any of the constitutive promoter such as pCS1, published in WO2014139608.

Specific examples of inducible or repressible promoter include promoters suitable for expressing polynucleotides in *A. niger,* such as *e.g.*, ptet-on (Meyer et al., Appl Environ Microbiol. 77(9):2975-83.) pgas (Yin et al. 2017, Appl Environ Microbiol.;83(6)), pbphA (Antunes et al, 2016, Appl Microbiol Biotechnol.;100(12):5479-89) pterA (Geib et al. 2017, Fungal Biol Biotechnol. 4:13.), pCatR (Sharma et al. 2012, FEMS Microbiol Lett.; 327(1):33-40), pgaaA/C (Kuivanen et al. 2015, Microb Cell Fact.;14:2) and functional variants thereof. Further exemplary promoters are the native pAOX1 or pAOX2 and functional variants thereof, any of the regulatory promoter, such as pG1-pG8, and fragments thereof, published in WO2013050551; any of the regulatory promoter, such as pG1 and pG1-x, published in WO2017021541 A1.

Suitable promoter sequences for use with yeast host cells are specifically described in Mattanovich et al. (Methods Mol. Biol. (2012) 824:329-58) and include glycolytic enzymes like triosephosphate isomerase (TPI), phosphoglycerate kinase (PGK), glyceraldehyde-3- phosphate dehydrogenase (GAPDH or GAP) and variants thereof, lactase (LAC) and galactosidase (GAL), *P. pastoris* glucose-6-phosphate isomerase promoter (PPGI), the 3- phosphoglycerate kinase promoter (PPGK), the glycerol aldehyde phosphate dehydrogenase promoter (pGAP), translation elongation factor promoter (PTEF), and the promoters of *P. pastoris* enolase 1 (PEN01), triose phosphate isomerase (PTPI), ribosomal subunit proteins (PRPS2, PRPS7, PRPS31, PRPL1), alcohol oxidase promoter (PAOX1, PAOX2) or variants thereof with modified characteristics, the formaldehyde dehydrogenase promoter (PFLD), isocitrate lyase promoter (PICL), alpha-ketoisocaproate decarboxylase promoter (PTHI), the promoters of heat shock protein family members or chaperones (PSSA1, PHSP90, PKAR2, PPDI1, PERO1), 6-Phosphogluconate dehydrogenase (PGND1), phosphoglycerate mutase (PGPM1), transketolase (PTKL1), phosphatidylinositol synthase (PPIS1), ferro-02-oxidoreductase (PFET3), high affinity iron permease (PFTR1), repressible alkaline phosphatase (PPH08), N-myristoyl transferase (PNMT1), pheromone response transcription factor (PMCM1), ubiquitin (PUBI4), single- stranded DNA endonuclease (PRAD2), the promoter of the major ADP/ATP carrier of the mitochondrial inner membrane (PPET9) (WO2008/128701) and the formate dehydrogenase (FMD) promoter. The GAP promoter, AOX1 or AOX2 promoter or a promoter derived from GAP or AOX1 or AOX2 promoter is particularly preferred. AOX promoters can be induced by methanol and are repressed by glucose.

Further examples of suitable promoters include *Saccharomyces cerevisiae* enolase (pENO-1), *Saccharomyces cerevisiae* galactokinase (pGAL1), *Saccharomyces cerevisiae* alcohol dehydrogenase/glyceraldehyde-3-phosphate dehydrogenase (pADH1, pADH2/pGAP), *Saccharomyces cerevisiae* triose phosphate isomerase (TPI), *Saccharomyces cerevisiae* metallothionein (pCUP1), and *Saccharomyces cerevisiae* 3-phosphoglycerate kinase (pPGK), and the maltase gene promoter (pMAL).

Specifically, one or more heterologous nucleic acid sequences may be incorporated into the host cell genome by any suitable technique of targeted or random genetic engineering. Specifically, the host cell genome comprises a heterologous *cexA* gene as a result of such genetic engineering. Specifically, the heterologous cexA gene is incorporated into a host cell chromosome, or into a plasmid.

According to a specific aspect, the expression cassette is integrated within a chromosome of the host cell, or within a plasmid. An expression cassette may be introduced into the host cell and integrated into the host cell genome as intrachromosomal element *e.g*., at a specific site of integration or randomly integrated, whereupon a high producer host cell line is selected. Alternatively, the expression cassette may be integrated within an extrachromosomal genetic element, such as a plasmid or YAC. According to a specific example, the expression cassette is introduced into the host cell by a vector, in particular an expression vector, which is introduced into the host cell by a suitable transfection or transformation technique. For this purpose, the POI encoding polynucleotide or gene may be ligated into an expression vector.

A preferred yeast expression vector (which is preferably used for expression in yeast) is selected from the group consisting of plasmids derived from pPICZ, pGAPZ, pPIC9, pPICZalfa, pGAPZalfa, pPIC9K, pGAPHis or pPUZZLE.

A preferred expression vector suitably used in filamentous fungi is any plasmid containing the AMA1 sequence (Sarkari et al. 2017) or derivatives thereof.

According to a specific example, a wild-type host cell may first be modified to introduce one or more expression cassettes for activating the *cexA* gene as further described herein, *e.g.*, an expression cassette expressing the *cexA* gene, as further described herein. Such modified host cell may then be further engineered to comprise an expression cassette for POI production.

According to another specific example, a wild-type host cell may first be engineered to comprise an expression cassette for POI production. Such engineered host cell may be further modified to introduce one or more expression cassettes for activating the *cexA* gene as further described herein, *e.g.*, an expression cassette expressing a *cexA* gene, as further described herein.

According to another specific example, the host cell may undergo one or more further genetic modifications *e.g*., for improving production of a fermentation product.

Techniques for transfecting or transforming prokaryotic or eukaryotic cells and for introducing a vector or plasmid are well-known in the art. These can include lipid vesicle mediated uptake, heat shock mediated uptake, calcium phosphate mediated transfection (calcium phosphate/DNA co-precipitation), microinjection and electroporation.

Specific embodiments refer to host cells which are engineered for overexpressing an endogenous *cexA* gene, or enhancing the CexA protein level or function in said host cell, or to insert a heterologous (*e.g.*, an artificial) polynucleotide encoding the *cexA* gene, or to host cells, wherein the *cexA* gene or CexA protein is otherwise activated.

According to a specific aspect, the modification activates the *cexA* gene resulting in increasing the level or activity of CexA, preferably wherein the modification up-regulates the *cexA* gene expression in said cell.

Activation of the *cexA* gene may include expression of one or more copies of the *cexA* gene as described herein, or the expression or activation of a transcription regulator, which regulates the expression of the *cexA* gene, a respective transcription activator, promoter, enhancer, or an inhibitor. Specifically, the transcription regulator is selected from regulatory polynucleotides capable of inducing increased *cexA* gene expression or function. Thereby a strain is obtained with increased organic acid export rate, in particular an increased citric acid export rate.

Specifically, the genetic modification comprises a gain-of-function alteration in the *cexA* gene, preferably wherein the host cell comprises one or more genetic modifications of the host cell genome comprising a disruption, substitution, insertion or knockin of (i) one or more heterologous polynucleotides, or a part thereof; or (ii) an expression control sequence of said one or more heterologous polynucleotides.

Specifically, the engineered host cell is overexpressing the *cexA* gene by such *cexA* gene activation. Overexpressing in a host cell which naturally produces and exports citric acid, may lead to an increase in the production rate of producing and exporting citric acid.

Specifically, the host cell is capable of at least any of 10%, 15%, 20%, 25%, or 30% increase in the production rate of producing citric acid relative to the host cell without said modification. Specifically, the production rate is determined by the amount obtained in the cell culture medium.

In a specific embodiment, the host cell is not naturally exporting citric acid into the cell culture medium, and further engineered to increase citric acid production and/or export of citric acid into the cell culture medium or supernatant. In such case, the host cell may comprise a modification for *cexA* gene activation.

Upon *cexA* gene expression or activation, the host cell is specifically capable of at least any of 10%, 15%, 20%, 25%, or 30% increase in the production rate of producing citric acid relative to the host cell without said modification for *cexA* gene activation.

The production rate may be determined as the host cell's specific productivity for citric acid (µg/g host cell dry mass (DM) per hour and/or volumetric productivity for citric acid (µg/g DM per hour).

Upon *cexA* gene expression or activation, the host cell is specifically capable of at least any of 5%, 10%, 15%, 20%, 25%, or 30% increase in the citric acid titer, as determined by the amount of citric acid in the cell culture medium (w/v).

Specifically, if a heterologous *e.g*., an artificial, *cexA* gene is used in a host cell, such *cexA* gene is considered active and functional, if it is capable of expressing a protein which increases the yield of citric acid obtained in the cell culture medium by at least any one of 10%, 15%, 20%, 25%, or 30%, as compared to the citric acid production without such heterologous *cexA* gene.

According to a specific aspect, the invention further provides for a method of producing citric acid or a salt thereof, by culturing the host cell as described herein, in particular the host cell engineered for *cexA* gene expression or activation, under conditions that permit the host cell to produce citric acid and to export citric acid, followed by isolating the produced citric acid or a salt thereof, from the cell culture.

Specific further embodiments refer to host cells which are engineered for downmodulating a *cexA* gene, or downmodulating a CexA protein level or function in said host cell, in particular an endogenous *cexA* gene or CexA protein, or to insert a heterologous or an artificial polynucleotide for inactivation of a *cexA* gene or CexA protein, or to host cells, wherein the *cexA* gene or CexA protein is otherwise inactivated.

According to a specific aspect, the modification inactivates the *cexA* gene resulting in reducing the level or activity of CexA, preferably wherein the modification down-regulates of eliminates the *cexA* gene expression in said cell.

Inactivation of the *cexA* gene may include one or more mutations inhibiting the expression of the *cexA* gene, or the expression or activation of a transcription regulator, which regulates the expression of the *cexA* gene, or a respective transcription inhibitor. Specifically, the transcription regulator is selected from regulatory polynucleotides capable of inducing reduced *cexA* gene expression or function. Thereby a strain is obtained with reduced organic acid export rate, in particular a reduced citric acid export rate. Particular embodiments refer to frame-shift mutations, deletions, or the knock-out of the *cexA* gene.

Specifically, the genetic modification comprises a loss-of-function alteration in the *cexA* gene, preferably wherein the host cell comprises one or more genetic modifications of the host cell genome comprising a disruption, substitution, deletion or knockout of (i) one or more endogenous polynucleotides, or a part thereof; or (ii) an expression control sequence of said one or more endogenous polynucleotides.

Specifically, the engineered host cell is reducing expression of the *cexA* gene or the level or activity of the CexA protein by such *cexA* gene inactivation. Reducing or inhibiting citric acid export to the cell culture medium may lead to an increase in the production rate of fermentation products which include *e.g.*, a protein of interest (POI) or cell metabolites other than citric acid.

Specifically, the host cell is capable of at least any of 10%, 15%, 20%, 25%, or 30% increase in the production rate of producing a fermentation product relative to the host cell without said modification for modulated *cexA* expression.

Specifically, the host cell is a *A. niger* strain comprising the endogenous *cexA* gene, which is genetically modified to express one or more further copies of the same *cexA* gene, or suitable artificial *cexA* genes, thereby increasing the export of citric acid into the host cell culture medium. In such case, citric acid or its salt (a citrate) can be isolated from the cell culture medium or supernatant at a high yield.

In a specific embodiment, the host cell is naturally secreting citric acid into the cell culture medium, and engineered to inhibit citric acid export into the cell culture medium. In such case, the host cell may comprise a modification for *cexA* gene inactivation.

Upon *cexA* gene inactivation, the host cell is specifically capable of at least any of 10%, 15%, 20%, 25%, or 30% increase in the production rate of producing a fermentation product other than citric acid relative to the host cell without said modification for *cexA* gene inactivation.

Upon *cexA* gene inactivation, the host cell is specifically capable of at least any of 5%, 10%, 15%, 20%, 25%, or 30% increase in the fermentation product titer, as determined by the amount of the fermentation product in the cell culture medium or supernatant (w/v).

Specific host cells described herein which comprise a *cexA* gene inactivation are provided as *cexA* deletion strains, including *e.g., cexA* deletion, knockdown or knockout mutations.

According to a specific aspect, the invention further provides for a method of producing a fermentation product other than citric acid by culturing the host cell as described herein, in particular the host cell engineered for *cexA* gene inactivation, under conditions that permit the host cell to produce the fermentation product, and isolating the produced fermentation product. According to a specific aspect, the method further comprises the step of isolating and/or purifying the fermentation product. Specifically, a purified fermentation product preparation is obtained that is isolated from the host cell culture medium or supernatant.

Specifically, the host cell comprising inactivated *cexA* produces a fermentation product which is
a) a cellular metabolite, preferably selected from the group consisting of erythritol, itaconic acid, aconitic acid, succinic acid, 2-hydroxyparaconic acid, itatartaric acid, pyruvic acid, isocitric acid, alpha ketoglutaric acid, malic acid, oxalic acid, gluconic acid, arabitol, xylitol, fumaric acid, acetic acid, and lactic acid; or
b) a protein of interest (POI), preferably a peptide or protein selected from the group consisting of therapeutic proteins, enzymes and peptides, protein antibiotics, toxin fusion proteins, carbohydrate - protein conjugates, structural proteins, regulatory proteins, vaccines, vaccine like proteins or particles, process enzymes, growth factors, hormones, and cytokines.

A specific aspect refers to the use of the host cell described herein, *e.g.*, any host cell comprising activated or inactivated *cexA,* in a method of producing a fermentation product, which method comprises culturing said host cell under conditions that permit the host cell to produce the fermentation product, and isolating the produced fermentation product.

Specifically, the fermentation product is a cellular metabolite, preferably selected from the group consisting of citric acid (in particular, if the host cell comprises activated *cexA*), erythritol, itaconic acid, aconitic acid, succinic acid, 2-hydroxyparaconic acid, itatartaric acid, pyruvic acid, isocitric acid, alpha ketoglutaric acid, malic acid, oxalic acid, gluconic acid, arabitol, xylitol, fumaric acid, acetic acid, and lactic acid.

Specifically, the method of producing fermentation products employs one or more of the following culturing conditions:
- The carbon source concentration is higher than 50 g L-1, preferably at least 200 g L-1.
- A carbon source is preferred, which enables a fast metabolism through glycolysis preferably glucose, fructose, maltose, saccharose.
- pH during the production phase is preferably below pH 3, when producing citric acid. At the beginning of the cultivation the pH can be above pH 5. Addition of a nitrogen source (*e.g*., ammonium sulfate, ammonium nitrate) leading to a pH decline below pH 2 within 48 hours is preferred.
- Aeration is in excess leading to a dissolved oxygen concentration of at least 10% preferably 20% and higher.
- Phosphate concentration is suboptimal, *e.g.*, below 50 mM leading to a limitation during the cultivation.
- Trace metal ions are preferably limited, *e.g*., the manganese (Mn²⁺) concentration is below 10 µg L-1, preferably below 2 µg L-1. For other trace elements like Cu²⁺, Mg²⁺, Fe²⁺, Zn²⁺ and Mo²⁺ ions, the concentrations are preferably lower than 10 ppm, more preferably lower than 1 ppm.

When culturing the host cell for metabolite production, the following modified Vogels medium without a Mn source is preferably used: 2.5 g/L Na₃-citrate 2×H₂O, 5 g/L KH₂PO₄,2g/L NH₄NO₃, 0.2 g/L MgSO₄ 7×H₂O, 0.1 g/L CaCl₂ 2×H₂O, 0.5 mg/L citric acid 1×H₂O, 0.5 mg/L ZnSO₄ 7×H₂O, 0.1 mg/L Fe(NH₄)₂ (SO₄)₂ 6×H₂O, 0.025 mg/L CuSO₄ 5×H₂O, 0.005 mg/L MnSO₄ 1×H₂O, 0.005 mg/L H₃BO₃, 0.005 mg/L Na₂MoO₄ 2×H₂O, and 0.005 mg/L biotin; pH is preferably adjusted to a starting pH of 5.5, strains are cultivated at temperatures between 30 and 35°C under continuous aeration, cultures are inoculated with conidia at a concentration of 10^5 to 10^8 conidia per liter.

Specifically, the fermentation product is a protein of interest (POI), in particular a recombinant POI. The POI can be any one of eukaryotic, prokaryotic or synthetic peptides, polypeptide, or proteins. Specifically, the POI is heterologous to the host cell species.

Specifically, the POI is a peptide, polypeptide or protein selected from the group consisting of therapeutic proteins, enzymes and peptides, protein antibiotics, toxin fusion proteins, carbohydrate - protein conjugates, structural proteins, regulatory proteins, vaccines, vaccine like proteins or particles, process enzymes, growth factors, hormones, and cytokines.

Specifically, the POI is a therapeutic or diagnostic product, preferably selected from the group consisting of antibody molecules or antigen-binding antibody fragments, antibody mimetics, fusion proteins, enzymes, cytokines, growth factors, clotting factors, hormones, pharmaceutical drug substances and vaccines.

The host cell described herein may suitably comprise one or more further genomic alterations to produce said fermentation product under culturing conditions, e.g., by incorporating one or more heterologous polynucleotides encoding a heterologous POI, a heterologous metabolite (such as produced by a metabolic pathway which may be at least partially heterologous), or facilitating the metabolite production.

According to a specific example, the erythritol production is increased by further modifying the host cell to overexpress a *gldB* gene encoding a NADP+-dependent glycerol dehydrogenase which has at least 70% sequence identity to SEQ ID NO:3.

Specifically, the glycerol dehydrogenase has at least any one of 70%, 80%, 90%, or 95% sequence identity to SEQ ID NO:3, and is functional as a NADP+-dependent glycerol dehydrogenase.

According to a specific aspect, the glycerol dehydrogenase is endogenous or heterologous to the host cell, preferably wherein the glycerol dehydrogenase has at least 70% sequence identity to SEQ ID NO:3.

Specifically, the glycerol dehydrogenase comprises or consists of SEQ ID NO:3. Specifically, the glycerol dehydrogenase is of *A. niger* origin, such as of wild-type *A. niger,* or an artificial protein obtainable by mutagenesis of such naturally occurring glycerol dehydrogenase of *A. niger* origin, which comprises one or more mutations in the amino acid sequence, and has any one of 70%, 80%, 90%, or 95% sequence identity to SEQ ID NO:3.

According to a specific aspect, the *gldB* gene is endogenous or heterologous to the host cell, preferably wherein the *gldB* gene has at least 70% sequence identity to SEQ ID NO:4.

Specifically, the *gldB* gene has at least any one of 70%, 80%, 90%, or 95% sequence identity to SEQ ID NO:4.

Specifically, the *gldB* gene is endogenous to the host cell, in particular naturally occurring in the wild-type host cell.

Specifically, the *gldB* gene comprises or consists of SEQ ID NO:4. Specifically, the *gldB* gene is of *A. niger* origin, or an artificial polynucleotide or gene obtainable by mutagenesis of such naturally occurring *gldB* gene of *A. niger* origin, which comprises one or more mutations in the nucleic acid sequence, and has any one of 70%, 80%, 90%, or 95% sequence identity to SEQ ID NO:4.

According to a specific embodiment, the host cell comprises one or more modifications, including modification(s) for *cexA* gene inactivation, and for *gldB* gene overexpression. A particular embodiment refers to an engineered *A. niger* host cell, which comprises at least one modification to inactivate the *cexA* gene, and at least one further modification to overexpress the *gldB* gene in said host cell.

For *gldB* gene overexpression, any of the methods and means for activating or overexpressing the *cexA* gene as described herein may be used, yet applied to the *gldB* gene. Such host cell may particularly be used in the production of cell metabolites, such as erythritol.

According to a specific example, itaconic acid production by the host cell is increased by further modifying the host cell to overexpress any one or more of *mttA, cadA* and *mfsA* genes.

The *mttA* gene encodes a mitochondrial tricarboxylic transporter (MTT) which has at least 70% sequence identity to SEQ ID NO:5, preferably at least any one of 70%, 80%, 90%, or 95% sequence identity to SEQ ID NO:5. The *mttA* gene described herein has at least 70% sequence identity to SEQ ID NO:6, preferably at least any one of 70%, 80%, 90%, or 95% sequence identity to SEQ ID NO:6, specifically which encodes a mitochondrial tricarboxylic transporter.

The *cadA* gene encodes a cis-aconitate decarboxylase (CAD) which has at least 70% sequence identity to SEQ ID NO:7, preferably at least any one of 70%, 80%, 90%, or 95% sequence identity to SEQ ID NO:7. The *cadA* gene described herein has at least 70% sequence identity to SEQ ID NO:8, preferably at least any one of 70%, 80%, 90%, or 95% sequence identity to SEQ ID NO:8, specifically which encodes a cis-aconitate decarboxylase.

The *mfsA* gene encodes the putative MFS (Major Facilitator Superfamily) transporter which has at least 70% sequence identity to SEQ ID NO:9, preferably at least any one of 70%, 80%, 90%, or 95% sequence identity to SEQ ID NO:9. The *mfsa* gene described herein has at least 70% sequence identity to SEQ ID NO:10, preferably at least any one of 70%, 80%, 90%, or 95% sequence identity to SEQ ID NO:10, specifically which encodes a protein with similar function as the putative MFS.

According to a specific aspect, any or each of the *mttA, cadA* and *mfsA* genes and the respective proteins is endogenous or heterologous to the host cell.

Specifically, the protein encoded by any of the *mttA, cadA* and *mfsA* genes is of *A. niger* origin, or an artificial protein obtainable by mutagenesis of such naturally occurring protein of *A. niger* origin, which comprises one or more mutations in the amino acid sequence, and has any one of 70%, 80%, 90%, or 95% sequence identity to the protein sequence as naturally produced by wild-type *A. niger.*

According to a specific aspect, any or each of the *mttA, cadA* and *mfsA* genes is endogenous or heterologous to the host cell, preferably wherein each of the *mttA, cadA* and *mfsA* genes overexpressed in the host cell has at least 70% sequence identity to the gene sequence which is naturally occurring in wild-type *A. niger.*

Specifically, any or each of the *mttA, cadA* and *mfsA* genes is endogenous to the host cell, in particular naturally occurring in the wild-type host cell.

According to a specific embodiment, the host cell comprises one or more modifications, including modification(s) for *cexA* gene inactivation, and for overexpression of any of each of the *mttA, cadA* and *mfsA* genes. A particular embodiment refers to an engineered *A. niger* host cell, which comprises at least one modification to inactivate the *cexA* gene, and at least one further modification to overexpress any or each of the *mttA*, *cadA* and *mfsA* genes in said host cell.

For gene overexpression, any of the methods and means for activating or overexpressing the *cexA* gene as described herein may be used, yet applied to any or each of the *mttA*, *cadA* and *mfsA* genes. Such host cell may particularly be used in the production of cell metabolites, such as itaconic acid.

The invention further provides for in a method of producing a fermentation product using the host cell described herein. Specifically, said method produces a fermentation product such as a POI or a metabolite, *e.g*., citric acid, its salt (a citrate e.g., sodium citrate), or any other cell metabolite, by culturing the host cell under conditions that permit production of said fermentation product. Specifically, the method comprises production and optionally secretion of the fermentation product into the cell culture medium, followed by isolating and optionally purifying said fermentation product. If the fermentation is a secreted product, e.g. citric acid or other metabolites exported into the cell culture medium, or a secreted POI, the fermentation product is suitably obtained from the cell culture medium.

Specifically, the host cell is a cell line cultured in a cell culture, in particular a production host cell line.

According to a specific embodiment, the cell line is cultured under batch, fed-batch or continuous culture conditions. The culture may be performed in microtiter plates, shake-flakes, or a bioreactor starting with a batch phase as the first step, followed by a fed-batch phase or a continuous culture phase as the second step.

According to a specific aspect, the invention further provides for a method of increasing the amount of a fermentation product produced by an engineered host cell described herein.

It was surprisingly found that the *cexA* gene was an essential citrate export gene in *A. niger.* Homologous genes are found in each organism comprising a citric acid cycle and metabolic pathways employing citric acid. Modulating the *cexA* gene expression improves the production of fermentation products. Modulation of gene expression may either refer to activation or inactivation, such as to express higher or lower amounts of activities of the CexA protein, as necessary for improving production processes.

With the genetic information available, overexpression of this CexA could be accomplished to enhance the citric acid or citrate production by *A. niger* strains. Furthermore, by heterologous expression of the *cexA* transporter gene, citric acid or citrate could also be produced by other host cells or microorganisms, by culturing in a cell culture, and isolating form the cell culture supernatant.

According to a specific example, overexpression of *cexA* gene in *A. niger* improved citric acid export and yields obtained in the cell culture medium.

According to another specific example, production of erythritol was improved in a citric acid free system using a *cexA* deletion in *A. niger.*

Yet, according to another specific example, overexpression of *gldB* in *A. niger* in order to further enhance erythritol formation was performed in a deletion strain of *cexA.*

Yet, according to another specific example, deletion of *cexA* and overexpression of *mttA*, *cadA* and *mfsA* genes was performed to produce itaconic acid in *A. niger.*

Yet, according to another specific example, heterologous expression of the *A. niger* citrate export gene (*cexA*) was successfully performed in *Saccharomyces cerevisiae* for metabolite production.

### FIGURES

Figure 1: Citric acid titers measured during the cultivation of a parental *A. niger* strain (ACIB1) compared to the *cexA* mutant strains K710_2; K710_12; K710_37 using Vogel's medium supplemented with 200 g/L glucose as carbon source, as described in the Examples.
Figure 2: Erythritol formed by A. niger strains (K710_2; K710_12; K710_37) having CexA deleted cultivated on Vogel's medium, as described in the Examples.

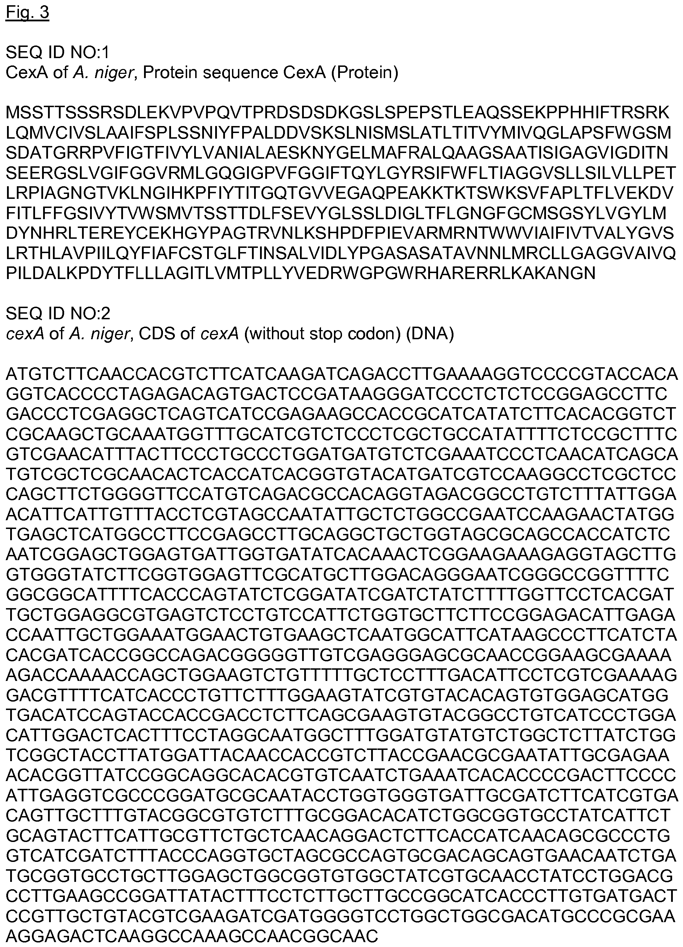

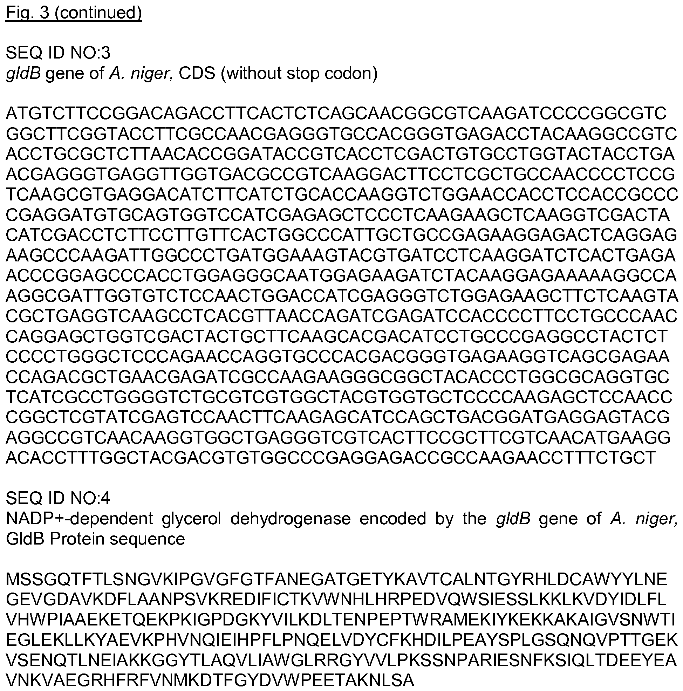

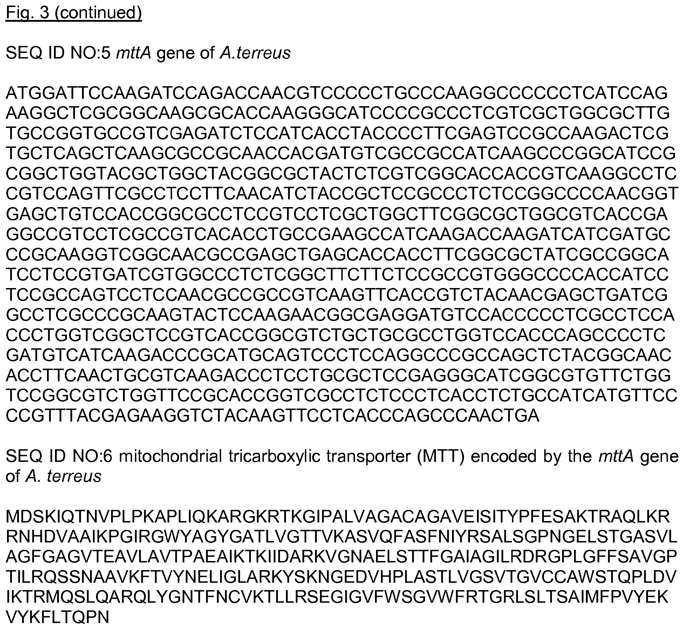

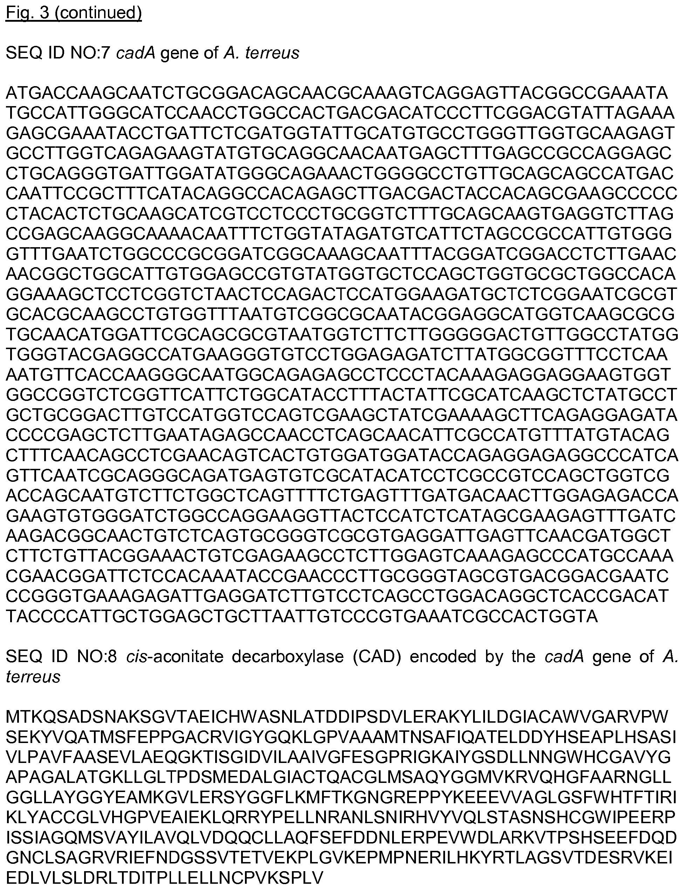

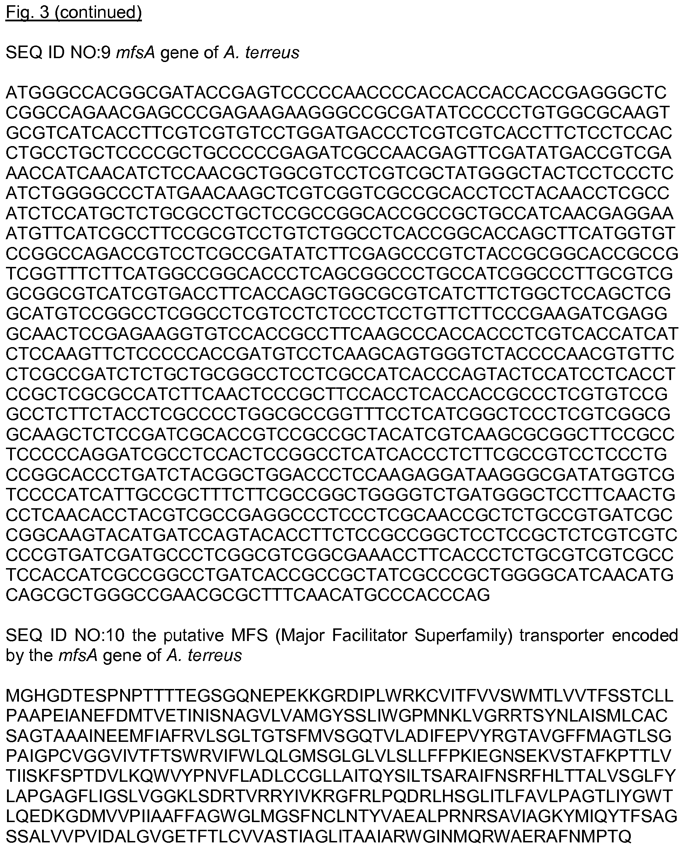

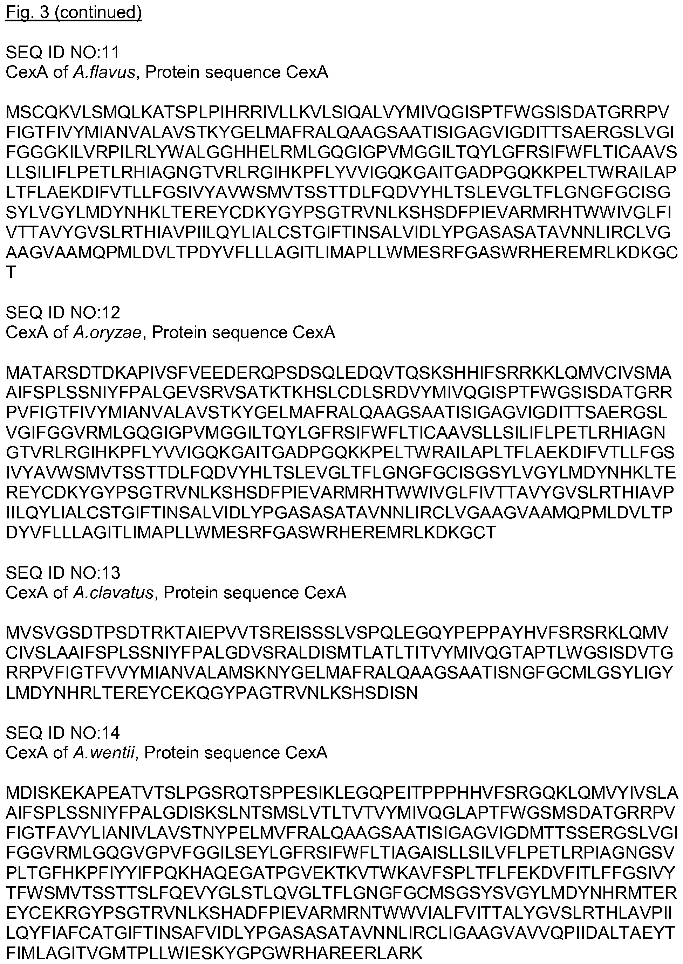

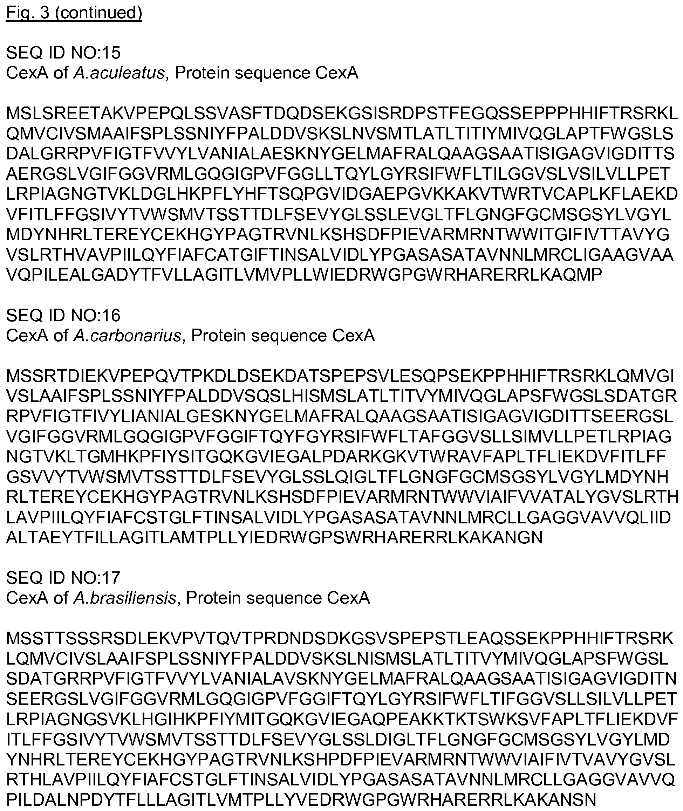

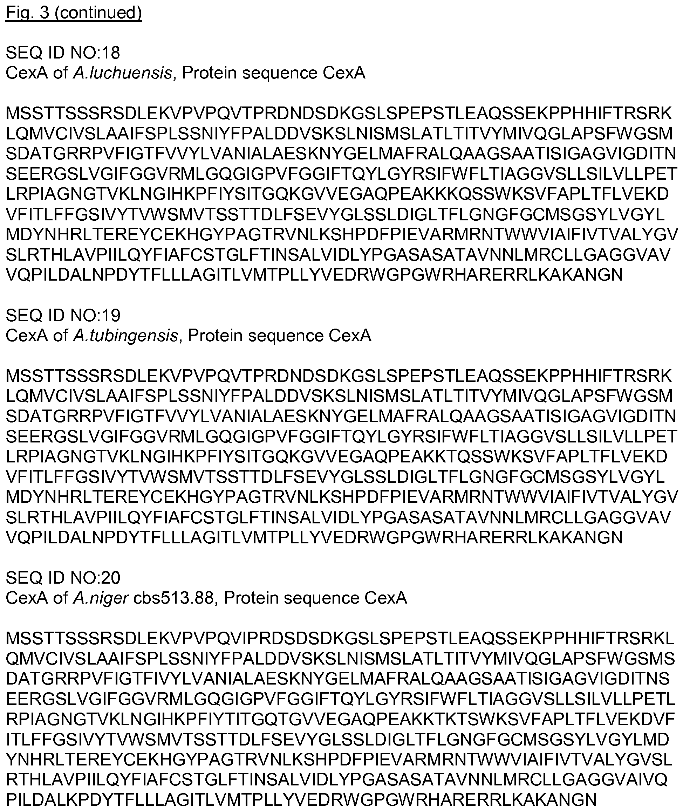

Figure 3: Sequences described herein
   SEQ ID NO:1 (CexA of *A. niger*).
   SEQ ID NO:2. (*cexA* of *A. niger*), CDS (without stop codon)
   SEQ ID NO:3 *gldB* gene of *A. niger,* CDS (without stop codon)
   SEQ ID NO:4 NADP+-dependent glycerol dehydrogenase encoded by the *gldB* gene of *A. niger,* GldB Protein sequence
   SEQ ID NO:5 *mttA* gene of *A. terreus*
   SEQ ID NO:6 mitochondrial tricarboxylic transporter (MTT) encoded by the *mttA* gene of *A. terreus*
   SEQ ID NO:7 *cadA* gene of *A. terreus*
   SEQ ID NO:8 *cis*-aconitate decarboxylase (CAD) encoded by the *cadA* gene of *A. terreus*
   SEQ ID NO:9 *mfsA* gene of *A. terreus*
   SEQ ID NO:10 the putative MFS (Major Facilitator Superfamily) transporter encoded by the *mfsA* gene of *A. terreus*

### Exemplary CexA homologs:

SEQ ID NO:11 CexA of *A.flavus*, Protein sequence CexA
SEQ ID NO:12 CexA of *A.oryzae*, Protein sequence CexA
SEQ ID NO:13 CexA of *A.clavatus,* Protein sequence CexA
SEQ ID NO:14 CexA of *A.wentii,* Protein sequence CexA
SEQ ID NO:15 CexA of *A.aculeatus,* Protein sequence CexA
SEQ ID NO:16 CexA of *A.carbonarius,* Protein sequence CexA
SEQ ID NO:17 CexA of *A.brasiliensis*, Protein sequence CexA
SEQ ID NO:18 CexA of *A.luchuensis*, Protein sequence CexA
SEQ ID NO:19 CexA of *A.tubingensis*, Protein sequence CexA
SEQ ID NO:20 CexA of *A.niger* CBS 513.88, Protein sequence CexA

### DETAILED DESCRIPTION OF THE INVENTION

Specific terms as used throughout the specification have the following meaning.

The term "host cell" as used herein shall refer to a single cell, a single cell clone, or a cell line of a host cell.

The term "cell line" as used herein refers to an established clone of a particular cell type that has acquired the ability to proliferate over a prolonged period of time. A cell line is typically used for expressing an endogenous or recombinant gene, or products of a metabolic pathway to produce polypeptides or cell metabolites mediated by such polypeptides. A "production host cell line" or "production cell line" is commonly understood to be a cell line ready-to-use for cell culture in a bioreactor to obtain the product of a production process, such as a fermentation product, which may be a cellular metabolite, either a primary or secondary metabolite, or a POI.

The host cell producing a fermentation product as described herein is also referred to as "production host cell", and a respective cell line a "production cell line".

The term "cell culture" as used herein with respect to a host cell refers to the maintenance of cells in an artificial, *e.g*., an *in vitro* environment, under conditions favoring growth, differentiation or continued viability, in an active or quiescent state, of the cells, specifically in a controlled bioreactor according to methods known in the industry.

When culturing a cell culture using appropriate culture media, the cells are brought into contact with the media in a culture vessel or with substrate under conditions suitable to support culturing the cells in the cell culture. In various embodiments described herein, a culture medium is provided that can be used for the growth of host cells. Standard cell culture techniques are well-known in the art.

The cell cultures as described herein particularly employ techniques which provide for the production of a fermentation product, which is exported such as to obtain the fermentation product in the cell culture medium, herein referred to as "cell culture supernatant", which is separable from the cellular biomass, and may be purified to obtain the fermentation product at a higher degree of purity. The purified fermentation product is particularly obtained upon separation of any of the co-expressed proteins or metabolites.

Cell culture media provide the nutrients necessary to maintain and grow cells in a controlled, artificial and *in vitro* environment. Characteristics and compositions of the cell culture media vary depending on the particular cellular requirements. Important parameters include osmolality, pH, and nutrient formulations. Feeding of nutrients may be done in a continuous or discontinuous mode according to methods known in the art.

Whereas a batch process is a cell culture mode in which all the nutrients necessary for culturing the cells are contained in the initial culture medium, without additional supply of further nutrients during fermentation, in a fed-batch process, after a batch phase, a feeding phase takes place in which one or more nutrients are supplied to the culture by feeding. Although in most processes the mode of feeding is critical and important, the host cell and methods described herein are not restricted with regard to a certain mode of cell culture.

In certain embodiments, the cell culture process is a fed-batch process. Specifically, a host cell transformed with a nucleic acid construct encoding a desired fermentation product, is cultured in a growth phase medium and transitioned to a production phase medium in order to produce a desired fermentation product.

In another embodiment, host cells described herein are cultured in a continuous mode, *e.g*., a chemostat. A continuous fermentation process is characterized by a defined, constant and continuous rate of feeding of fresh culture medium into a bioreactor, whereby culture broth is at the same time removed from the bioreactor at the same defined, constant and continuous removal rate. By keeping culture medium, feeding rate and removal rate at the same constant level, the cell culture parameters and conditions in the bioreactor remain constant.

A fermentation product can be produced using the host cell and the respective cell line described herein, by culturing in an appropriate medium, isolating the expressed fermentation product from the culture, and optionally purifying it by a suitable method.

Several different approaches for the production of a fermentation product as described herein are preferred. A fermentation product may be produced by expressing, processing and optionally exporting or secreting the product from a host cell, comprising transforming the host cell with an expression vector harboring recombinant DNA encoding the relevant protein, in particular a POI or a relevant protein of a metabolic pathway, preparing a culture of the transformed cell, growing the culture, inducing transcription and production of the fermentation product, and recovering the fermentation product.

The host cell described herein may be tested for its capability to export organic acids, in particular citric acid, and further for its production capacity by the yield of fermentation product obtained from a cell culture, by any of the following tests: ELISA, activity assay, HPLC, Western blot, MS or other suitable tests.

To determine the effect of a modification for the host cell's modulated *cexA* gene expression, the increase, or reduction, or even elimination of citric acid transport can be determined by suitable means of determining the amount of citric acid in the cell culture supernatant. For this purpose, the host cell line may be cultured in microtiter plates, shake flask, or bioreactor using fedbatch or chemostat fermentations in comparison with a host cell line without such modification in the respective cell.

The production method described herein specifically allows for the fermentation on a pilot or industrial scale. The industrial process scale would preferably employ volumina of at least 10 L, specifically at least 50 L, preferably at least 1 m³, preferably at least 10 m³, most preferably at least 100 m³.

Production conditions in industrial scale are preferred, which refer to e.g., fed batch culture in reactor volumes of 100 L to 10 m³ or larger, employing typical process times of several days, or continuous processes in fermenter volumes of approximately 50 - 1000 L or larger, with dilution rates of approximately 0.02 - 0.15 h⁻¹.

In specific embodiments, the cells produce and export metabolites into the cell culture medium, including primary and secondary metabolites, such as citric acid or other metabolites including *e.g*., sugar alcohols, polyols, or organic acids. Exemplary metabolites are citric acid, erythritol, itaconic acid, aconitic acid, succinic acid, 2-hydroxyparaconic acid, itatartaric acid, pyruvic acid, isocitric acid, alpha ketoglutaric acid, malic acid, oxalic acid, gluconic acid, arabitol, xylitol, fumaric acid, acetic acid, and lactic acid.

The metabolite is preferably produced employing conditions to obtain yields of at least 100 mg/L, preferably at least 1000 mg/L, preferably at least 10 g/L, most preferred at least 100 g/L.

In specific further embodiments, the cells express or produce a product, such as a recombinant therapeutic or diagnostic product. As described in more detail below, examples of products produced by cells include, but are not limited to, POIs such as exemplified herein including antibody molecules (e.g., monoclonal antibodies, bispecific antibodies), antibody mimetics (polypeptide molecules that bind specifically to antigens but that are not structurally related to antibodies such as e.g. DARPins, affibodies, adnectins, or IgNARs), fusion proteins (e.g., Fc fusion proteins, chimeric cytokines), other recombinant proteins (e.g., glycosylated proteins, enzymes, hormones), or viral therapeutics (e.g., anti-cancer oncolytic viruses, viral vectors for gene therapy and viral immunotherapy), cell therapeutics (e.g., pluripotent stem cells, mesenchymal stem cells and adult stem cells), vaccines or lipid-encapsulated particles (e.g., exosomes, virus-like particles), RNA (such as e.g. siRNA) or DNA (such as e.g. plasmid DNA), antibiotics or amino acids.

The POI is preferably expressed employing conditions to produce yields of at least 1 mg/L, preferably at least 10 mg/L, preferably at least 100 mg/L, most preferred at least 1 g/L.

The term "CexA" as used herein refers to the CexA protein of *A. niger* which comprises or consists of SEQ ID NO:1, and/or which is encoded by the *cexA* gene, but shall also refer to any other natively occurring or artificial proteins which have at least any one of 70%, 80%, 90%, or 95% sequence identity to SEQ ID NO:1, and which promote the export of organic acids, such as citric acid, in a host cell producing such CexA protein. The CexA protein may be of natural origin, e.g. naturally occurring in a prokaryotic or eukaryotic organism, or artificial, such as a functional mutant or variant with at least any one of 70%, 80%, 90%, or 95% sequence identity to the naturally-occurring sequence. The CexA function is specifically characterized by the function as organic acid export protein. Specific CexA proteins are orthologs or paralogs of the CexA protein of *A. niger.*

The term "*cexA* gene" as used herein refers to the *cexA* gene of *A. niger* which comprises or consists of SEQ ID NO:2, but shall also refer to any other natively occurring or artificial polynucleotides or gene(s) which have at least any one of 70%, 80%, 90%, or 95% sequence identity to SEQ ID NO:2, and which encode a protein that promotes the export of organic acids, such as citric acid, in a host cell expressing such *cexA* gene. The *cexA* gene may be of natural origin, e.g. naturally occurring in a prokaryotic or eukaryotic organism, or artificial, such as a functional mutant or variant with at least any one of 70%, 80%, 90%, or 95% sequence identity to the naturally-occurring sequence. The *cexA* function variant is specifically characterized by the capability to functional CexA.

In some embodiments, the functional variant of a polynucleotide or nucleic acid molecule comprises a nucleotide sequence which is sequence optimized e.g., for improving nucleic acid stability, increasing translation efficacy in the host cell, reducing the number of truncated proteins expressed, improving the folding or prevent misfolding of the expressed proteins, reducing toxicity of the expressed products, reducing cell death caused by the expressed products, or increasing and/or decreasing protein aggregation. According to a specific embodiment, the functional variant of a parent nucleotide sequence is a codon-optimized variant of said parent nucleotide sequence to be expressed in a host cell, which is obtainable by one or more modifications of the parent nucleotide sequence for improved expression in the cellular environment of the host cell.

A functionally active variant can be prepared by mutagenesis of a native (wild-type) polynucleotide or nucleic acid molecule to produce a variant thereof, expressing the variant in the host cell, and assessing the activity of the variant to express its function e.g., by measuring the level or activity of the encoded protein.

Functional variants of a parent protein include, for instance, proteins wherein one or more amino acid residues are added, or deleted, at the N-or C-terminus, as well as within one or more internal domains. Specific functionally active variant comprise additional amino acids at the N-terminal and/or at the C-terminal end, to prolong a parent sequence, e.g. by less than 100 amino acids, specifically less than 75 amino acids, more specifically less than 50 amino acids, more specifically less than 25 amino acids, or else less than 10 amino acids. Further specific functionally active variants may be fusion proteins, wherein a sequence is prolonged by additional amino acid residues of another polypeptide or protein.

Specific functional variants are fragments of a parent protein or nucleic acid molecule.

Functional variants which are fragments of a polynucleotide or nucleic acid molecule may comprise or consist of at least 50% of the respective nucleotide sequence, preferably at least any of 60, 70, 80 or 90%.

Functional variants which are fragments of a polypeptide or protein may comprise or consist of at least 10 amino acids, specifically at least 25 amino acids, more specifically at least 50 amino acids, more specifically at least 75 amino acids, or at least 100 contiguous amino acids, or up to the total number of amino acids present in a full-length protein.

The term "endogenous" as used herein is meant to include those molecules and sequences, in particular endogenous polynucleotides, genes, polypeptides or proteins, which are present in the wild-type (native) host cell. In particular, an endogenous nucleic acid molecule (*e.g*., a gene) or protein that does occur in a particular host cell as it is found in nature, is understood to be "host cell endogenous" or "endogenous to the host cell". Moreover, a cell "endogenously expressing" a nucleic acid or protein expresses that nucleic acid or protein as does a host of the same particular type as it is found in nature. Moreover, a host cell "endogenously producing" or that "endogenously produces" a nucleic acid, protein, or other compound produces that nucleic acid, protein, or compound as does a host cell of the same particular type as it is found in nature.

The term "heterologous" as used herein with respect to a nucleotide or amino acid sequence or protein, refers to a compound which is either foreign, *i.e.* "exogenous", such as not found in nature, to a given host cell; or that is naturally found in a given host cell, *e.g*., is "endogenous", however, in the context of a heterologous construct, *e.g*., employing a heterologous nucleic acid, thus "not naturally-occurring". The heterologous nucleotide sequence as found endogenously may also be produced in an unnatural, *e.g*., greater than expected or greater than naturally found, amount in the cell. The heterologous nucleotide sequence, or a nucleic acid comprising the heterologous nucleotide sequence, possibly differs in sequence from the endogenous nucleotide sequence but encodes the same protein as found endogenously. Specifically, heterologous nucleotide sequences are those not found in the same relationship to a host cell in nature (*i.e.*, "not natively associated"). Any recombinant or artificial nucleotide sequence is understood to be heterologous. An example of a heterologous polynucleotide or nucleic acid molecule comprises a nucleotide sequence not natively associated with a promoter, *e.g*., to obtain a hybrid promoter, or operably linked to a coding sequence, as described herein. As a result, a hybrid or chimeric polynucleotide may be obtained. A further example of a heterologous compound is a POI encoding polynucleotide or gene operably linked to a transcriptional control element, *e.g*., a promoter, to which an endogenous, naturally-occurring POI coding sequence is not normally operably linked.

The term "export" in relation to fermentation products as used herein shall mean the secretion and/or transport or otherwise passaging of such products through the cellular wall, thereby obtaining the product outside the cell, typically in a cell culture medium or supernatant.

The term "expression cassette" as used herein refers to nucleic acid molecules containing a desired coding sequence and control sequences in operable linkage, so that hosts transformed or transfected with these sequences are capable of producing the encoded proteins or host cell metabolites. In order to effect transformation, the expression system may be included in a vector; however, the relevant DNA may also be integrated into a host chromosome. Expression may refer to secreted or non-secreted expression products, particularly those exported into the cell culture medium, including e.g., polypeptides or metabolites.

The terms "express" and "expression" as used herein shall mean allowing or causing the information in a gene, RNA or DNA sequence to become manifest; for example, producing a protein by activating the cellular functions involved in transcription and translation of a corresponding gene. A DNA sequence is expressed in or by a cell to form an "expression product" such as an RNA (e.g., mRNA) or a protein. The expression product itself may also be said to be "expressed" by the cell.

The term "expression" in particular means the transcription of a gene or genes or genetic construct into structural RNA (rRNA, tRNA) or mRNA with or without subsequent translation of the latter into a protein. The process includes transcription of DNA and processing of the resulting mRNA product. The term "expression" or "gene expression" can also include the translation of the mRNA and therewith the synthesis of the encoded protein, i.e., protein expression.

Expression cassettes are conveniently provided as expression constructs *e.g*., in the form of "vectors" or "plasmids", which are typically DNA sequences that are required for the transcription of cloned recombinant nucleotide sequences, *i.e.* of recombinant genes and the translation of their mRNA in a suitable host organism. Expression vectors or plasmids usually comprise an origin for autonomous replication in the host cells, selectable markers (*e.g*., an amino acid synthesis gene or a gene conferring resistance to antibiotics such as zeocin, kanamycin, G418, nourseothricin or hygromycin), a number of restriction enzyme cleavage sites, a suitable promoter sequence and a transcription terminator, which components are operably linked together. The terms "plasmid" and "vector" as used herein include autonomously replicating nucleotide sequences as well as genome integrating nucleotide sequences, such as artificial chromosomes *e.g*., a yeast artificial chromosome (YAC).

Expression vectors may include but are not limited to cloning vectors, modified cloning vectors and specifically designed plasmids. Preferred expression vectors described herein are expression vectors suitable for expressing of a recombinant gene in a eukaryotic host cell and are selected depending on the host organism. Appropriate expression vectors typically comprise regulatory sequences suitable for expressing DNA encoding a desired protein in a host cell. Examples of regulatory sequences include operators, enhancers, ribosomal binding sites, and sequences that control transcription and translation initiation and termination. The regulatory sequences may be operably linked to the DNA sequence to be expressed.

To allow expression of a recombinant nucleotide sequence in a host cell, the expression vector may provide a promoter adjacent to the 5' end of the coding sequence, *e.g.*, upstream from a gene of interest or a signal peptide gene enabling secretion of the protein encoded by such gene of interest. The transcription is thereby regulated and initiated by this promoter sequence.

The expression construct described herein specifically comprises a promoter operably linked to a nucleotide sequence encoding a desired protein, such as CexA or a POI, under the transcriptional control of said promoter. Specifically, the promoter is not natively associated with the coding sequence of the protein.

Also multicloning vectors, which are vectors having a multicloning site, can be used as described herein, wherein a desired heterologous gene can be incorporated at a multicloning site to provide an expression vector. In the case of multicloning vectors, because the gene is introduced at the multicloning site, a promoter is typically placed upstream of the multicloning site.

The term "modulated expression" as used herein shall refer to the increased or decreased expression of a polynucleotide or gene. Modulation particularly means in relation to expression or gene expression, a process in which the expression level is changed by said gene expression in comparison to the control host, the expression level may be increased or decreased. The original, unmodulated expression may be of any kind of expression of a structural RNA (rRNA, tRNA) or mRNA with subsequent translation. For the purposes described herein, the original unmodulated expression may also be absence of any expression. The term "modulating expression" with respect to the proteins or nucleic acids used in the methods, constructs, expression cassettes, vectors, plants, seeds, host cells and uses described herein shall mean any change of the expression of the respective nucleic acid sequences or encoded proteins. The expression can increase from zero (absence of, or immeasurable expression) to a certain amount, or can decrease from a certain amount to immeasurable small amounts or zero.

The term "activate" in relation to a gene, as used herein means any form of expression that is higher than in the comparable host, without such activated gene *e.g.*, a wild-type host. Gene activation may lead to overexpression.

The term "overexpression" as used herein means any form of expression that is additional to the comparable or original wild-type expression level. For the purposes described herein, the original wild-type expression level might also be zero, i.e. absence of expression or immeasurable expression. Reference herein to "upregulated expression" means increased expression, enhanced expression or overexpression, and is meant to refer to an increase in gene expression and/or, as far as referring to polypeptides, increased polypeptide levels and/or increased polypeptide activity, relative to control hosts. The increase in expression, polypeptide levels or polypeptide activity is in increasing order of preference at least 10%, 20%, 30%, 40% or 50%, 60%, 70%, 80%, 85%, 90%, or 100% or even more compared to that of control hosts. In cases when the control hosts have only very little expression, the increase in expression, polypeptide levels or polypeptide activity may be even higher, such as at least 100 times, 200 times, 300 times, 400 times, 500 times, 600 times, 700 times, 800 times, 900 times, 1000 times, 2000 times, 3000 times, 5000 times, 10 000 times, 20 000 times, 50 000 times, 100 000 times or even more compared to that of control hosts.

Methods for increasing expression of genes or gene products are well documented in the art and include, for example, insertion of at least one heterologous expression cassette comprising and expressing the gene into the host, or overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to increase expression of a nucleic acid encoding the polypeptide of interest. For example, endogenous promoters may be altered *in vitro* or *in vivo* by mutation, deletion, and/or substitution, or isolated promoters may be introduced into a host cell in the proper orientation and distance from a gene so as to control the expression of the gene.

The term "inactivate" in relation to a gene, as used herein means any form of expression that is lower *i.e*., a decreased expression, than in the comparable host, without such inactivated gene *e.g*., a wild-type host. Gene inactivation may lead to down-regulation of a gene, herein also referred to as down-modulation.

The term "decreased expression" as used herein, shall mean inhibition or reduction or substantial elimination of expression, and is meant as a a decrease in endogenous gene expression and/or polypeptide levels and/or polypeptide activity relative to control hosts. The reduction or substantial elimination is in increasing order of preference at least 10%, 20%, 30%, 40% or 50%, 60%, 70%, 80%, 85%, 90%, or 95%, 96%, 97%, 98%, 99% or more compared to that of control hosts.

Gene expression of a gene may be inhibited or reduced by methods which directly interfere with gene expression, encompassing, but not restricted to, inhibition or reduction of DNA transcription, *e.g.*, by use of specific promoter-related repressors, by site specific mutagenesis of a given promoter, by promoter exchange, or inhibition or reduction of translation, *e.g.*, by RNAi induced post-transcriptional gene silencing. The expression of a dysfunctional, or inactive gene product with reduced activity, can, for example, be achieved by site specific or random mutagenesis, insertions or deletions within the coding gene.

The inhibition or reduction of the activity of gene product can, for example, be achieved by administration of, or incubation with, an inhibitor to the respective enzyme, prior to or simultaneously with protein expression. Examples for such inhibitors include, but are not limited to, an inhibitory peptide, an antibody, an aptamer, a fusion protein or an antibody mimetic against said enzyme, or a ligand or receptor thereof, or an inhibitory peptide or nucleic acid, or a small molecule with similar binding activity. Other ways to inhibit the enzyme are the reduction of specific cofactors of the enzyme in the medium, like copper, which is a PAM specific ion cofactor (*e.g.*, in the form of CuSO₄), ascorbate, which acts as an electron donor for PAM, molecular oxygen, catalase and others known today to the skilled artisan, or yet to be discovered in the future.

Gene deletion strains typically comprise elements or genetic modifications for gene silencing, gene knock-down and gene knockout, resulting in significant reduction or elimination of gene expression. Such gene deletion strains may be produced by techniques by which the expression of a gene is reduced, either through genetic modification or by treatment with an oligonucleotide with a sequence complementary to either an mRNA transcript or a gene. If genetic modification of DNA is done, the result is a knock-down or knockout organism. If the change in gene expression is caused by an oligonucleotide binding to an mRNA or temporarily binding to a gene, this results in a temporary change in gene expression without modification of the chromosomal DNA and is referred to as a transient knock-down.

It is well understood that inactivation of the *cexA* gene in a host cell, such as in a *cexA* deletion strain, interfers with citric acid export of the host cell, but does not hinder the ability to produce citric acid intracellularly.

In a transient knock-down, which is also encompassed by the above term, the binding of this oligonucleotide to the active gene or its transcripts causes decreased expression through blocking of transcription (in the case of gene-binding), degradation of the mRNA transcript (*e.g*., by small interfering RNA (siRNA) or RNase-H dependent antisense) or blocking either mRNA translation, pre-mRNA splicing sites or nuclease cleavage sites used for maturation of other functional RNAs such as miRNA (*e.g*., by Morpholino oligos or other RNase-H independent antisense). Other approaches involve the use of shRNA (small hairpin RNA, which is a sequence of RNA that makes a tight hairpin turn that can be used to silence gene expression via RNA interference), esiRNA (Endoribonuclease-prepared siRNAs, which are a mixture of siRNA oligos resulting from cleavage of long double-stranded RNA (dsRNA) with an endoribonuclease), or the activation of the RNA-induced silencing complex (RISC).

Other approaches to carry out gene silencing, knock-down or knockout are known to the skilled person from the respective literature, and their application in the context of the present invention is considered as routine. Gene knockout refers to techniques by which the expression of a gene is fully blocked, *i.e.* the respective gene is inoperative, or even removed. Methodological approaches to achieve this goal are manifold and known to the skilled person. Examples are the production of a mutant which is dominantly negative for the given gene. Such mutant can be produced by site directed mutagenesis (*e.g*., deletion, partial deletion, insertion or nucleic acid substitution), by use of suitable transposons, or by other approaches which are known to the skilled person from the respective literature, the application of which in the context of the present invention is thus considered as routine. One example is knockout by use of targeted Zinc Finger Nucleases. A respective Kit is provided by Sigma Aldrich as "CompoZR knockout ZFN". Another approach encompasses the use of Transcription activator-like effector nucleases (TALENs).

The delivery of a dominant negative construct involves the introduction of a sequence coding for a dysfunctional enzyme, *e.g*., by transfection. Said coding sequence is functionally coupled to a strong promoter, in such way that the gene expression of the dysfunctional enzyme overrules the natural expression of the wild type enzyme, which, in turn, leads to an effective physiological defect of the respective enzyme activity.

A conditional gene knockout allows blocking gene expression in a tissue- or time-specific manner. This is done, for example, by introducing short sequences called IoxP sites around the gene of interest. Again, other approaches are known to the skilled person from the respective literature, and their application in the context of the present invention is considered as routine.

One other approach is gene alteration which may lead to a dysfunctional gene product or to a gene product with reduced activity. This approach involves the introduction of frame shift mutations, nonsense mutations (*i.e.*, introduction of a premature stop codon) or mutations which lead to an amino acid substitution which renders the whole gene product dysfunctional, or causing a reduced activity. Such gene alteration can for example be produced by mutagenesis (*e.g.*, deletion, partial deletion, insertion or nucleic acid substitution), either unspecific (random) mutagenesis or site directed mutagenesis. Protocols describing the practical application of gene silencing, gene knock-down, gene knockout, delivery of a dominant negative construct, conditional gene knockout, and/or gene alteration are commonly available to the skilled artisan, and are within his routine. The technical teaching provided herein is thus entirely enabled with respect to all conceivable methods leading to an inhibition or reduction of gene expression of a gene product, or to the expression of a dysfunctional, or inactive gene product, or with reduced activity.

The host cell as described herein specifically refers to an artificial organism and a derivative of a native (wild-type) host cell. It is well understood that the host cells, methods and uses described herein, *e.g*., specifically referring to one or more (genetic) modifications, expression constructs, transformed host cells and recombinant proteins, are non-naturally occurring, "man-made" or synthetic, and are therefore not considered as a "natural product" or a result of "law of nature".

Genetic modifications described herein may employ tools, methods and techniques known in the art, such as described by J. Sambrook et al., Molecular Cloning: A Laboratory Manual (3rd edition), Cold Spring Harbor Laboratory, Cold Spring Harbor Laboratory Press, New York (2001).

The term "metabolite" as used herein refers to an organic compound which is synthetized by a host cell, *i.e.* a biosynthetic product. The metabolite can be synthesized via natural and/or genetically modified metabolism including both intracellular and extracellular products that can be either primary or secondary metabolites. Specific metabolites are by-products or final products of a metabolic pathway, including *e.g.*, small molecule secondary metabolites, polyketides, fatty acid synthase products, non-ribosomal peptides, ribosomal peptides, proteins and enzymes.

The term "primary metabolite" as used herein, refers to a product produced intracellularly or extracellularly by a host cell *e.g*., a microorganism, during the growth phase including metabolites involved in growth, development and reproduction that typically are energy metabolism related products such as citric acid, ethanol and lactic acid are primary metabolites.

The term "secondary metabolite" refers to compounds that are not directly involved in the normal growth, development, or reproduction of an organism. Secondary metabolites are often restricted to a narrow set of species within a phylogenetic group and often play an important role in defense systems. Certain secondary metabolites are used as colorings, flavorings and medicines. Examples of secondary metabolites are alkaloids (such as atropine, cocaine, codeine, morphine, tetrodotoxin), natural phenols (such as polyphenols), monoterpenoids (such as geranyl diphosphate, limonene, pinene), diterpenoids (such as aphidicolin, geranylgeranyl diphosphate, pimaradiene, taxol), NRP's, pigments (such as chrysogenin), mycotoxins (such as roquefortin) and antibiotics (such as a β-lactam like 6-aminopenicillanic acid, 7-aminodesacetoxycephalosporanic acid, adipyl-7-aminodesacetoxycephalosporanic acid, cephalosporin C, penicillin G or penicillin V, streptomycin, tetracyclin).

Some of the metabolites referred to herein are organic acids *e.g.*, citric acid. It is well understood that the term "metabolite" not only refers to the organic acid directly produced by the host cell, but also to salts thereof which may be present in the cell culture medium or produced when isolating or purifying the metabolite. Therefore, when describing "citric acid" as a fermentation product, such term shall also encompass "citrates".

The term "promoter" as used herein refers to a DNA sequence capable of controlling the expression of a coding sequence or functional RNA. Promoter activity may be assessed by its transcriptional efficiency. This may be determined directly by measurement of the amount of mRNA transcription from the promoter, e.g. by Northern Blotting or indirectly by measurement of the amount of gene product expressed from the promoter.

The term "operably linked" as used herein refers to the association of nucleotide sequences on a single nucleic acid molecule, e.g. a vector, in a way such that the function of one or more nucleotide sequences is affected by at least one other nucleotide sequence present on said nucleic acid molecule. For example, a promoter is operably linked with a coding sequence of a recombinant gene, when it is capable of effecting the expression of that coding sequence. As a further example, a nucleic acid encoding a signal peptide is operably linked to a nucleic acid sequence encoding a protein, when it is capable of expressing a protein in the secreted form, such as a preform of a mature protein or the mature protein. Specifically, such nucleic acids operably linked to each other may be immediately linked, i.e. without further elements or nucleic acid sequences in between the nucleic acid encoding the signal peptide and the nucleic acid sequence encoding a protein.

A promoter sequence is typically understood to be operably linked to a coding sequence, if the promoter controls the transcription of the coding sequence. If a promoter sequence is not natively associated with the coding sequence, its transcription is either not controlled by the promoter in native (wild-type) cells or the sequences are recombined with different contiguous sequences.

The term "protein of interest (POI)" as used herein refers to a polypeptide or a protein that is produced by means of recombinant technology in a host cell. More specifically, the protein may either be a polypeptide not naturally occurring in the host cell, *i.e.* a heterologous protein, or else may be native to the host cell, *i.e.* a homologous protein to the host cell, but is produced, for example, by transformation with a self-replicating vector containing the nucleic acid sequence encoding the POI, or upon integration by recombinant techniques of one or more copies of the nucleic acid sequence encoding the POI into the genome of the host cell, or by recombinant modification of one or more regulatory sequences controlling the expression of the gene encoding the POI, *e.g.*, of the promoter sequence.

The term "recombinant" as used herein shall mean "being prepared by or the result of genetic engineering. A recombinant host may be engineered to delete and/or inactivate one or more nucleotides or nucleotide sequences, and may specifically comprise an expression vector or cloning vector contain a recombinant nucleic acid sequence, in particular employing nucleotide sequence foreign to the host. A recombinant protein is produced by expressing a respective recombinant nucleic acid in a host.

The host cell described herein specifically comprises genetic modifications which are the result of genetic engineering. In such case, the host cell is meant to be a recombinant host cell.

The term "recombinant" with respect to a POI as used herein, includes a POI that is prepared, expressed, created or isolated by recombinant means, such as a POI isolated from a host cell transformed to express the POI. In accordance with the present invention conventional molecular biology, microbiology, and recombinant DNA techniques within the skill of the art may be employed. Such techniques are explained fully in the literature. See, *e.g*., Maniatis, Fritsch & Sambrook, "Molecular Cloning: A Laboratory Manual, Cold Spring Harbor, (1982).

The term "sequence identity" of a variant, homolog or ortholog as compared to a parent or comparable nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%.

Sequence similarity searches can identify such homologous proteins or genes by detecting excess similarity, and statistically significant similarity that reflects common ancestry. Homologues may encompass orthologues, which are herein understood as the same protein in different organisms, *e.g*., variants of such protein in different different organisms or species.

A homologous or orthologous sequence of the same protein in different organisms or species, specifically of the same genus, typically has at least about 50% sequence identity, preferably at least about 60% identity, more preferably at least about 70% identity, more preferably at least about 80% identity, more preferably at least about 90% identity, more preferably at least about 95% identity.

As described herein, homologous sequences of the CexA protein comprising or consisting of SEQ ID NO:1 are any of the following: CexA of *A.flavus* (SEQ ID NO:11), *A.oryzae* (SEQ ID NO:12), *A.clavatus* (SEQ ID NO:13), *A.wentii* (SEQ ID NO:14), *A.aculeatus* (SEQ ID NO:15), *A.carbonarius* (SEQ ID NO:16), *A.brasiliensis* (SEQ ID NO:17), *A.luchuensis* (SEQ ID NO:18), *A.tubingensis* (SEQ ID NO:19), or *A.niger* cbs513.88 (SEQ ID NO:20).

"Percent (%) amino acid sequence identity" with respect to an amino acid sequence, homologs and orthologues described herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

For purposes described herein, the sequence identity between two amino acid sequences is determined using the NCBI BLAST program version 2.2.29 (Jan-06-2014) with blastp set at the following exemplary parameters: Matrix: BLOSUM62; compositional adjustments: conditional compositional score matrix adjustment; Word Size: 6; Expect value: 10; Gap costs: Existence = 11 , Extension = 1; Filter = low complexity not activated.

"Percent (%) identity" with respect to a nucleotide sequence *e.g.*, of a promoter or a gene, is defined as the percentage of nucleotides in a candidate DNA sequence that is identical with the nucleotides in the DNA sequence, after aligning the sequence and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for measuring alignment, including any algorithms needed to achieve maximal alignment over the full length of the sequences being compared. For purposes described herein, the sequence identity between two nucleotide sequences is determined using the NCBI BLAST program version 2.2.29 (Jan-06-2014) with blastn set at the following exemplary parameters: Word Size: 28; Expect value: 10; Gap costs: linear; Filter = low complexity activated; Match/Mismatch Scores: 1,-2.

The term "isolated" or "isolation" as used herein with respect to a fermentation product shall refer to such compound that has been sufficiently separated from the environment with which it would naturally be associated, in particular a cell culture supernatant, so as to exist in "purified" or "substantially pure" form. Yet, "isolated" does not necessarily mean the exclusion of artificial or synthetic mixtures with other compounds or materials, or the presence of impurities that do not interfere with the fundamental activity, and that may be present, for example, due to incomplete purification. Isolated compounds can be further formulated to produce preparations thereof, and still for practical purposes be isolated - for example, a POI can be mixed with pharmaceutically acceptable carriers or excipients when used in diagnosis or therapy.

The term "purified" as used herein shall refer to a preparation comprising at least 50% (w/w), preferably at least 60%, 70%, 80%, 90% or 95% of a compound (*e.g*., a metabolite or a POI). Purity is measured by methods appropriate for the compound (*e.g*., chromatographic methods, polyacrylamide gel electrophoresis, HPLC analysis, and the like). An isolated, purified fermentation product as described herein may be obtained by purifying the cell culture supernatants to reduce impurities.

As isolation and purification methods for obtaining a recombinant polypeptide or protein product, methods, such as methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be used.

The following standard methods are preferred: cell (debris) separation and wash by Microfiltration or Tangential Flow Filter (TFF) or centrifugation, POI purification by precipitation or heat treatment, POI activation by enzymatic digest, POI purification by chromatography, such as ion exchange (IEX), hydrophobic interaction chromatography (HIC), Affinity chromatography, size exclusion (SEC) or HPLC Chromatography, POI precipitation of concentration and washing by ultrafiltration steps.

A highly purified product is essentially free from contaminating proteins, in particular from contaminating host cell protein, and preferably has a purity of at least 90%, more preferred at least 95%, or even at least 98%, up to 100%. The purified products may be obtained by purification of the cell culture supernatant or else from cellular debris.

An isolated and purified POI can be identified by conventional methods such as Western blot, HPLC, MS, activity assay, or ELISA.

An isolated and purified metabolite can be identified by conventional methods such as HPLC analysis, GC analysis, or enzymatic analysis.

Metabolites such as citric acid, erythritol, itaconic acid, aconitic acid, succinic acid, 2-hydroxyparaconic acid, itatartaric acid, pyruvic acid, isocitric acid, alpha ketoglutaric acid, malic acid, oxalic acid, gluconic acid, arabitol, xylitol, fumaric acid, acetic acid, and lactic acid, are advantageously isolated by precipitation and purified by recrystallization or chromatography-based methods.

The foregoing description will be more fully understood with reference to the following examples. Such examples are, however, merely representative of methods of practicing one or more embodiments of the present invention and should not be read as limiting the scope of invention.

### EXAMPLES

### Example 1: Inactivation of the citric acid transport gene (cexA) in Aspergillus niger

*Aspergillus niger* ACIB1 and ATCC 1015 were used as parental strains throughout this study. ACIB1 is an industrial derivative of ATCC 1015. *Escherichia coli* DH10B (Thermo Fisher Scientific, Waltham, USA) was used for plasmid propagation.

It was unexpectedly found that a high citric acid producing strain (ACIB1) has a particularly high transcription level of a gene named *cexA* (citrate export gene A) and the encoded protein CexA (citrate export protein A). This was unexpected because CexA was not amongst the putative citrate transporter candidates disclosed by Yin et al. (Sci. Rep. 2017, 7:1-16).

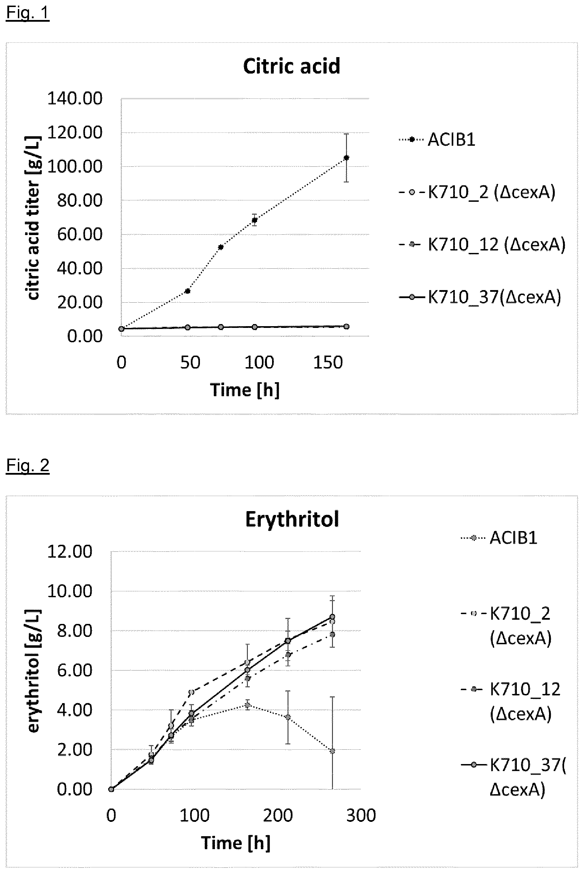

The coding sequence is provided as SEQ ID NO:2 and the amino acid sequence of the protein as SE ID NO:1. The transcript of this gene is about 60 times more abundant in the strain ACIB 1 compared to strain ATCC1015 measured by DNA microarrays (Agilent Technologies, Santa Clara, US). A bioinformatic analysis reveals that this gene encodes for a protein (Uniprot: A0A254TZW7_ASPNG), which contains transmembrane spanning helices and has the PROSITE annotation of a transport protein belonging to the class of major facilitator superfamily transporter (PS50850). According to this observation the gene *cexA* was mutated applying a CRISPR/Cas9 protocol (Sarkari et al., 2017) using the CRISPR targeting sequences (SEQ ID NO:21: CGTGTGAAGATATGATGCGG**TGG**) including the exemplary PAM site "TGG", which consists of the three nucleotides at the 3' end of SEQ ID NO:21 indicated in bold, to introduce deletion or insertion in the coding sequence of cexA. Mutant strains (K710_2; K710_12; K710_37) were selected, which contained mutations in the sequence targeted by the CRISPR gRNA leading to a frame shift in the ORF (open reading frame) of the gene leading to a truncated and inactive protein product. Cultivation of these mutant strains showed that these strains are not capable to secrete citric acid to the media when cultivated with glucose as carbon source, while the parental strain can secret more than 110 g/L under same cultivation conditions (Figure 1). Taken together these data show that cexA encodes a transport protein, which is responsible for the citrate secretion capability of *A. niger.*

### Example 2: Overexpression of cexA in Aspergillus niger strain ATCC1015 to improve citric acid secretion

### Strain generation and Media

*Aspergillus niger* ATCC 1015 was used as parental strains throughout this study. *Escherichia coli* DH10B was used for plasmid propagation.

The coding sequence (CDS) of the gene *cexA* (SEQ ID NO:2 and with a Stop codon added) is subcloned using a GoldenGate cloning procedure (Sarkari et al. 2017). Using the GoldenMOCS cloning procedure the CDS is fused with a promoter sequence, pmbfA (Blumhoff, M.L., Steiger, M.G., Marx, H., Mattanovich, D., Sauer, M., 2013. Six novel constitutive promoters for metabolic engineering of Aspergillus niger. Appl. Microbiol. Biotechnol. 97, 259-267. doi:10.1007/s00253-012-4207-9) and ptet-on (Meyer, V., Wanka, F., van Gent, J., Arentshorst, M., van den Hondel, C. a M.J.J., Ram, A.F.J., 2011. Fungal gene expression on demand: an inducible, tunable, and metabolism-independent expression system for Aspergillus niger. Appl. Environ. Microbiol. 77, 2975-83. doi:10.1128/AEM.02740-10), and a terminator sequence, ttrpC, yielding the functional expression cassettes for *A. niger:* pmbfA::cexA::ttrpC and ptet-on::cexA::ttrpC. Thereby, the cassette containing the pmbfA promoter is yielding a constitutive expression of CexA and the cassette with the ptet-on promoter is yielding a doxycycline inducible promoter. The resulting expression cassettes are cloned into a backbone 3 construct of the GoldenMOCS system in order to integrate the functional expression cassettes into the *pyrG* locus of *Aspergillus niger* (ATCC1015) as described by (Sarkari, P., Marx, H., Blumhoff, M.L., Mattanovich, D., Sauer, M., Steiger, M.G., 2017. An efficient tool for metabolic pathway construction and gene integration for Aspergillus niger. Bioresour. Technol. doi:10.1016/j.biortech.2017.05.004).

Protoplast transformation of *A. niger* is performed according to the previously described method (Arentshorst, M., Ram, A.F.J., Meyer, V., 2012. Using non-homologous end-joining-deficient strains for functional gene analyses in filamentous fungi. Methods Mol. Biol. 835, 133-50. doi:10.1007/978-1-61779-501-5_9). Selection of transformants is achieved on MMS plates. All transformants are purified by single colony isolation on the selection medium at least twice. The integration of the expression cassettes is checked by PCR after DNA extraction using primers specified to check integration on the pyrG locus (Sarkari, P., Marx, H., Blumhoff, M.L., Mattanovich, D., Sauer, M., Steiger, M.G., 2017. An efficient tool for metabolic pathway construction and gene integration for Aspergillus niger. Bioresour. Technol. doi:10.1016/j.biortech.2017.05.004). Overexpression strains are named either OE_pmbfA::cexA (containing the cassette pmbfA::cexA::ttrpC) or , OE_pteton::cexA (containing the cassette ptet-on::cexA::ttrpC). Strains are cultivated on MM plates for 5 days at 30°C to obtain conidia. Conidia are harvested, washed and counted as described.

Strains are cultivated on Vogel's medium (Blumhoff, M.L., Steiger, M.G., Mattanovich, D., Sauer, M., 2013a. Targeting enzymes to the right compartment: metabolic engineering for itaconic acid production by Aspergillus niger. Metab. Eng. 19, 26-32)] with 200 g/L glucose as carbon source to produce citric acid (Steiger, M.G., Punt, P.J., Ram, A.F.J.J., Mattanovich, D., Sauer, M., 2016. Characterizing MttA as a mitochondrial cis-aconitic acid transporter by metabolic engineering. Metab. Eng. 35, 95-104. doi:10.1016/j.ymben.2016.02.003). The parental strain ATCC1015 is compared to the overexpression strains (OE_pmbfA::cexA, OE_pteton::cexA). The strains with an inducible expression cassette (OE_pteton::cexA) are induced with doxycycline by adding doxycycline to the medium (1 µg doxycycline/mL and 10µg doxycyline/mL as described by (Steiger et al., 2016).

Metabolites (glucose, citric acid, erythritol) are quantified in the supernatant of the culture by HPLC analysis as described previously (Steiger, M.G., Punt, P.J., Ram, A.F.J.J., Mattanovich, D., Sauer, M., 2016. Characterizing MttA as a mitochondrial cis-aconitic acid transporter by metabolic engineering. Metab. Eng. 35, 95-104. doi:10.1016/j.ymben.2016.02.003).

### Example 3: Production of erythritol in a citric acid free Aspergillus niger system using a cexA deletion

In the citric acid producing strain ACIB1 the gene *cexA* is mutated using a CRISPR/Cas9 strategy. Cas9 is targeted by the gRNA to the target DNA sequence provided as SEQ ID NO 3 as described above. The resulting strains are cultivated as described in Example 2 on Vogel's medium with 200 g/L glucose. Metabolites are quantified in the supernatant of the culture as described in Example 2. Results of such an experiment are presented in Figure 2. The resulting strains produce higher titers of erythritol (up to 9 g/L) compared to the parental strain (ACIB 1) with an intact CexA protein (Figure 2). The parental strain produces about 4 g/L and then starts consuming the erythritol. No citric acid is production is observed in the strains expressing a mutated and inactivated CexA protein (K710_2, K710_12, K710_13) (Figure 1). Erythritol can be further isolated from the cultivation broth without the necessity to remove citric acid from the broth (Figure 2).

### Example 4: Heterologous expression of the A. niger citrate export gene (cexA) in Saccharomyces cerevisiae

The *cexA* coding sequence (SEQ ID NO:2 and equipped with at Stop codon for *S. cerevisiae*) is subcloned with the GoldenMOCS system (as described in Example 2) to produce a functional expression cassette in *S. cerevisiae.* For this purpose the yeast tpi1 promoter used as promoter and a cyc1 terminator yielding the constitutive expression cassette (ptpi1::cexA::tcyc1, Geertman, J.-M.A., van Dijken, J.P., Pronk, J.T., 2006. Engineering NADH metabolism in Saccharomyces cerevisiae: formate as an electron donor for glycerol production by anaerobic, glucose-limited chemostat cultures. FEMS Yeast Res. 6, 1193-1203. doi:10.1111/j.1567-1364.2006.00124.xis). The expression cassette is integrated into a Backbone 3 construct with a 2µ origin of replication and a hygromycin resistance cassette. *S. cerevisiae* strain CEN.PK (Maris, Antonius J.. Van, Winkler, A., Porro, D., Dijken, Johannes van, Pronk, J., 2004. Homofermentstive Lactate Production Cannot sustain anerobic growth of enginneered saccharomyes cerevisiea: possible consequences of engery dependent lactate export. Appl. envirmontal Microbiol. 70, 2898-2905. doi:10.1128/AEM.70.5.2898) is used as recipient and transformed as described by Gietz, R.D. and R.A. Woods. (Methods in Enzymology 2002, 350: 87-96) yielding CEN.PK + pCexA. Strains are propagated on YNB plates as described b and additionally supplemented with hygromycin. Strains are cultivated in shake flasks on medium containing glucose and ethanol as carbon source as described by Valli (Valli, M., Sauer, M., Branduardi, P., Borth, N., Porro, D., Mattanovich, D., 2006. Improvement of lactic acid production in Saccharomyces cerevisiae by cell sorting for high intracellular pH. Appl. Environ. Microbiol. 72, 5492-9. doi:10.1128/AEM.00683-06). Metabolites are quantified in the supernatant of the culture as described in Example 2.

### Example 5: Erythritol production in A. niger by overexpression of gldB in an A. niger deletion strain of cexA

In order to enhance the production of erythritol in *A. niger* a deletion mutant strain of *cexA* (from Example 1: K710_2) is transformed with a plasmid containing a functional expression cassette of the pmbfA promoter and trpC terminator as described in Example 2 fused to the *gldB* coding sequence (SEQ ID NO:3, and equipped with at Stop codon for *A. niger*). Integration is achieved at the pyrG locus of *A. niger* following the procedure described in Example 2. Strains (K710_2 + *OEgldB*) are cultivated as described in Example 2 and metabolites (erythritol, citric acid, glucose) are measured by HPLC as described in Example 2.

### Example 6: Deletion cexA and overexpression of mttA, cadA and mfsA gene to produce itaconic acid in Aspergillus niger

The coding sequences of the *A. terreus* genes *mttA, cadA* and *mfsA* (van der Straat, L., Vernooij, M., Lammers, M., van den Berg, W., Schonewille, T., Cordewener, J., van der Meer, I., Koops, A., de Graaff, L.H., 2014. Expression of the Aspergillus terreus itaconic acid biosynthesis cluster in Aspergillus niger. Microb. Cell Fact. 13, 11. doi:10.1186/1475-2859-13-11; Steiger, M.G., Punt, P.J., Ram, A.F.J.J., Mattanovich, D., Sauer, M., 2016. Characterizing MttA as a mitochondrial cis-aconitic acid transporter by metabolic engineering. Metab. Eng. 35, 95-104. doi:10.1016/j.ymben.2016.02.003) are subcloned with the GoldenMOCS system as described in Example 2 to yield expression cassettes controlled by the pmbfA promoter and ttrpC terminator (pmbfA::mtta::ttrpC; pmbfA::mfsa::ttrpC, pmbfA::cadA::ttrpC). The cassettes are integrated at the pyrG locus in the strain K710_2 following the procedure described in Example 2. Strains (K710_2 + OE*mttA, cadA, mfsA*) are cultivated as described in Example 2 and metabolites (citric acid, itaconic acid, erythritol, glucose, aconitic acid) are analyzed by HPLC.

## Claims

1. A host cell comprising a modification for modulated expression of a citrate export gene A (*cexA*), wherein the *cexA* gene encodes a protein which has at least 70% sequence identity to SEQ ID NO:1 (CexA).

2. The host cell of claim 1, which is a filamentous fungal cell, preferably Aspergillus, Trichoderma, Fusarium, Penicillium, or yeast, preferably Pichia, Yarrowia, Hansenula, Komagataella, or Saccharomyces.

3. The host cell of claim 1 or 2, wherein the *cexA* gene is endogenous or heterologous to the host cell, preferably wherein the *cexA* gene has at least 70% sequence identity to SEQ ID NO:2.

4. The host cell of any one of claims 1 to 3, wherein the modification is a genetic modification which results in at least one heterologous polynucleotide within the genome of the host cell.

5. The host cell of claim 4, wherein the heterologous polynucleotide is within an expression cassette expressing the *cexA* gene, preferably wherein the heterologous polynucleotide comprises a promoter and/or gene sequence, preferably wherein the cell has been transformed with said expression cassette.

6. The host cell of any one of claims 1 to 5, wherein the modification activates the *cexA* gene resulting in increasing the level or activity of CexA, preferably wherein the modification up-regulates the *cexA* gene expression in said cell.

7. The host cell of claim 6, which comprises a gain-of-function alteration in the *cexA* gene, preferably wherein the host cell comprises one or more genetic modifications of the host cell genome comprising a disruption, substitution, insertion or knockin of (i) one or more heterologous polynucleotides, or a part thereof; or (ii) an expression control sequence of said one or more heterologous polynucleotides.

8. The host cell of claim 6 or 7, which is capable of at least a 10% increase in the production rate of producing citric acid or a salt thereof, relative to the host cell without said modification.

9. A method of producing citric acid or a salt thereof, by culturing the host cell of any one of claims 1 to 8 under conditions that permit the host cell to produce citric acid, and isolating the produced citric acid or a salt thereof.

10. The host cell of any one of claims 1 to 5, wherein the modification inactivates the *cexA* gene resulting in reducing the level or activity of CexA, preferably wherein the modification down-regulates the *cexA* gene expression in said cell.

11. The host cell of claim 10, which comprises a loss-of-function alteration in the *cexA* gene, preferably wherein the host cell is comprises one or more genetic modifications of the host cell genome comprising a disruption, substitution, deletion or knockout of (i) one or more endogenous polynucleotides, or a part thereof; or (ii) an expression control sequence of said one or more endogenous polynucleotides.

12. The host cell of claim 10 or 11, which is capable of at least a 10% increase in the production rate of producing a fermentation product other than citric acid relative to the host cell without said modification.

13. A method of producing a fermentation product other than citric acid by culturing the host cell of any one of claims 10 to 12 under conditions that permit the host cell to produce the fermentation product, and isolating the produced fermentation product.

14. The method of claim 13, wherein the fermentation product is
a) a cellular metabolite, preferably selected from the group consisting of erythritol, itaconic acid, aconitic acid, succinic acid, 2-hydroxyparaconic acid, itatartaric acid, pyruvic acid, isocitric acid, alpha ketoglutaric acid, malic acid, oxalic acid, gluconic acid, arabitol, xylitol, fumaric acid, acetic acid, and lactic acid; or
b) a protein of interest (POI), preferably a peptide or protein selected from the group consisting of therapeutic proteins, enzymes and peptides, protein antibiotics, toxin fusion proteins, carbohydrate - protein conjugates, structural proteins, regulatory proteins, vaccines, vaccine like proteins or particles, process enzymes, growth factors, hormones, and cytokines.

15. Use of the host cell of any one of claims 1 to 8, or 10 to 12, in a method of producing a fermentation product, which method comprises culturing said host cell under conditions that permit the host cell to produce the fermentation product, and isolating the produced fermentation product.


SEQUENCE LISTING

<110>  ACIB GmbH
       Universität für Bodenkultur Wien

<120>  HOST CELL ENGINEERED FOR IMPROVED METABOLITE PRODUCTION

<130>  AC014EP

<160>  21

<170>  PatentIn version 3.5

<210>  1
<211>  524
<212>  PRT
<213>  Aspergillus niger

<400>  1

Met Ser Ser Thr Thr Ser Ser Ser Arg Ser Asp Leu Glu Lys Val Pro
1               5                   10                  15

Val Pro Gln Val Thr Pro Arg Asp Ser Asp Ser Asp Lys Gly Ser Leu
            20                  25                  30

Ser Pro Glu Pro Ser Thr Leu Glu Ala Gln Ser Ser Glu Lys Pro Pro
        35                  40                  45

His His Ile Phe Thr Arg Ser Arg Lys Leu Gln Met Val Cys Ile Val
    50                  55                  60

Ser Leu Ala Ala Ile Phe Ser Pro Leu Ser Ser Asn Ile Tyr Phe Pro
65                  70                  75                  80

Ala Leu Asp Asp Val Ser Lys Ser Leu Asn Ile Ser Met Ser Leu Ala
                85                  90                  95

Thr Leu Thr Ile Thr Val Tyr Met Ile Val Gln Gly Leu Ala Pro Ser
            100                 105                 110

Phe Trp Gly Ser Met Ser Asp Ala Thr Gly Arg Arg Pro Val Phe Ile
        115                 120                 125

Gly Thr Phe Ile Val Tyr Leu Val Ala Asn Ile Ala Leu Ala Glu Ser
    130                 135                 140

Lys Asn Tyr Gly Glu Leu Met Ala Phe Arg Ala Leu Gln Ala Ala Gly
145                 150                 155                 160

Ser Ala Ala Thr Ile Ser Ile Gly Ala Gly Val Ile Gly Asp Ile Thr
                165                 170                 175

Asn Ser Glu Glu Arg Gly Ser Leu Val Gly Ile Phe Gly Gly Val Arg
        180                 185                 190

Met Leu Gly Gln Gly Ile Gly Pro Val Phe Gly Gly Ile Phe Thr Gln
    195                 200                 205

Tyr Leu Gly Tyr Arg Ser Ile Phe Trp Phe Leu Thr Ile Ala Gly Gly
    210                 215                 220

Val Ser Leu Leu Ser Ile Leu Val Leu Leu Pro Glu Thr Leu Arg Pro
225                 230                 235                 240

Ile Ala Gly Asn Gly Thr Val Lys Leu Asn Gly Ile His Lys Pro Phe
                245                 250                 255

Ile Tyr Thr Ile Thr Gly Gln Thr Gly Val Val Glu Gly Ala Gln Pro
            260                 265                 270

Glu Ala Lys Lys Thr Lys Thr Ser Trp Lys Ser Val Phe Ala Pro Leu
        275                 280                 285

Thr Phe Leu Val Glu Lys Asp Val Phe Ile Thr Leu Phe Phe Gly Ser
    290                 295                 300

Ile Val Tyr Thr Val Trp Ser Met Val Thr Ser Ser Thr Thr Asp Leu
305                 310                 315                 320

Phe Ser Glu Val Tyr Gly Leu Ser Ser Leu Asp Ile Gly Leu Thr Phe
                325                 330                 335

Leu Gly Asn Gly Phe Gly Cys Met Ser Gly Ser Tyr Leu Val Gly Tyr
            340                 345                 350

Leu Met Asp Tyr Asn His Arg Leu Thr Glu Arg Glu Tyr Cys Glu Lys
        355                 360                 365

His Gly Tyr Pro Ala Gly Thr Arg Val Asn Leu Lys Ser His Pro Asp
    370                 375                 380

Phe Pro Ile Glu Val Ala Arg Met Arg Asn Thr Trp Trp Val Ile Ala
385                 390                 395                 400

Ile Phe Ile Val Thr Val Ala Leu Tyr Gly Val Ser Leu Arg Thr His
                405                 410                 415

Leu Ala Val Pro Ile Ile Leu Gln Tyr Phe Ile Ala Phe Cys Ser Thr
            420                 425                 430

Gly Leu Phe Thr Ile Asn Ser Ala Leu Val Ile Asp Leu Tyr Pro Gly
        435                 440                 445

Ala Ser Ala Ser Ala Thr Ala Val Asn Asn Leu Met Arg Cys Leu Leu
    450                 455                 460

Gly Ala Gly Gly Val Ala Ile Val Gln Pro Ile Leu Asp Ala Leu Lys
465                 470                 475                 480

Pro Asp Tyr Thr Phe Leu Leu Leu Ala Gly Ile Thr Leu Val Met Thr
                485                 490                 495

Pro Leu Leu Tyr Val Glu Asp Arg Trp Gly Pro Gly Trp Arg His Ala
            500                 505                 510

Arg Glu Arg Arg Leu Lys Ala Lys Ala Asn Gly Asn
        515                 520

<210>  2
<211>  1572
<212>  DNA
<213>  Aspergillus niger

<400>  2
atgtcttcaa ccacgtcttc atcaagatca gaccttgaaa aggtccccgt accacaggtc      60

acccctagag acagtgactc cgataaggga tccctctctc cggagccttc gaccctcgag     120

gctcagtcat ccgagaagcc accgcatcat atcttcacac ggtctcgcaa gctgcaaatg     180

gtttgcatcg tctccctcgc tgccatattt tctccgcttt cgtcgaacat ttacttccct     240

gccctggatg atgtctcgaa atccctcaac atcagcatgt cgctcgcaac actcaccatc     300

acggtgtaca tgatcgtcca aggcctcgct cccagcttct ggggttccat gtcagacgcc     360

acaggtagac ggcctgtctt tattggaaca ttcattgttt acctcgtagc caatattgct     420

ctggccgaat ccaagaacta tggtgagctc atggccttcc gagccttgca ggctgctggt     480

agcgcagcca ccatctcaat cggagctgga gtgattggtg atatcacaaa ctcggaagaa     540

agaggtagct tggtgggtat cttcggtgga gttcgcatgc ttggacaggg aatcgggccg     600

gttttcggcg gcattttcac ccagtatctc ggatatcgat ctatcttttg gttcctcacg     660

attgctggag gcgtgagtct cctgtccatt ctggtgcttc ttccggagac attgagacca     720

attgctggaa atggaactgt gaagctcaat ggcattcata agcccttcat ctacacgatc     780

accggccaga cgggggttgt cgagggagcg caaccggaag cgaaaaagac caaaaccagc     840

tggaagtctg tttttgctcc tttgacattc ctcgtcgaaa aggacgtttt catcaccctg     900

ttctttggaa gtatcgtgta cacagtgtgg agcatggtga catccagtac caccgacctc     960

ttcagcgaag tgtacggcct gtcatccctg gacattggac tcactttcct aggcaatggc 1020

tttggatgta tgtctggctc ttatctggtc ggctacctta tggattacaa ccaccgtctt 1080

accgaacgcg aatattgcga gaaacacggt tatccggcag gcacacgtgt caatctgaaa 1140

tcacaccccg acttccccat tgaggtcgcc cggatgcgca atacctggtg ggtgattgcg 1200

atcttcatcg tgacagttgc tttgtacggc gtgtctttgc ggacacatct ggcggtgcct 1260

atcattctgc agtacttcat tgcgttctgc tcaacaggac tcttcaccat caacagcgcc 1320

ctggtcatcg atctttaccc aggtgctagc gccagtgcga cagcagtgaa caatctgatg 1380

cggtgcctgc ttggagctgg cggtgtggct atcgtgcaac ctatcctgga cgccttgaag 1440

ccggattata ctttcctctt gcttgccggc atcacccttg tgatgactcc gttgctgtac 1500

gtcgaagatc gatggggtcc tggctggcga catgcccgcg aaaggagact caaggccaaa 1560

gccaacggca ac 1572

<210> 3
<211> 975
<212> DNA
<213> Aspergillus niger

<400> 3
atgtcttccg gacagacctt cactctcagc aacggcgtca agatccccgg cgtcggcttc 60

ggtaccttcg ccaacgaggg tgccacgggt gagacctaca aggccgtcac ctgcgctctt 120

aacaccggat accgtcacct cgactgtgcc tggtactacc tgaacgaggg tgaggttggt 180

gacgccgtca aggacttcct cgctgccaac ccctccgtca agcgtgagga catcttcatc 240

tgcaccaagg tctggaacca cctccaccgc cccgaggatg tgcagtggtc catcgagagc 300

tccctcaaga agctcaaggt cgactacatc gacctcttcc ttgttcactg gcccattgct 360

gccgagaagg agactcagga gaagcccaag attggccctg atggaaagta cgtgatcctc 420

aaggatctca ctgagaaccc ggagcccacc tggagggcaa tggagaagat ctacaaggag 480

aaaaaggcca aggcgattgg tgtctccaac tggaccatcg agggtctgga gaagcttctc 540

aagtacgctg aggtcaagcc tcacgttaac cagatcgaga tccacccctt cctgcccaac 600

caggagctgg tcgactactg cttcaagcac gacatcctgc ccgaggccta ctctcccctg 660

ggctcccaga accaggtgcc cacgacgggt gagaaggtca gcgagaacca gacgctgaac 720

gagatcgcca agaagggcgg ctacaccctg gcgcaggtgc tcatcgcctg gggtctgcgt 780

cgtggctacg tggtgctccc caagagctcc aacccggctc gtatcgagtc caacttcaag 840

agcatccagc tgacggatga ggagtacgag gccgtcaaca aggtggctga gggtcgtcac 900

ttccgcttcg tcaacatgaa ggacaccttt ggctacgacg tgtggcccga ggagaccgcc 960

aagaaccttt ctgct 975

<210>  4
<211>  325
<212>  PRT
<213>  Aspergillus niger

<400>  4

Met Ser Ser Gly Gln Thr Phe Thr Leu Ser Asn Gly Val Lys Ile Pro
1               5                   10                  15

Gly Val Gly Phe Gly Thr Phe Ala Asn Glu Gly Ala Thr Gly Glu Thr
            20                  25                  30

Tyr Lys Ala Val Thr Cys Ala Leu Asn Thr Gly Tyr Arg His Leu Asp
        35                  40                  45

Cys Ala Trp Tyr Tyr Leu Asn Glu Gly Glu Val Gly Asp Ala Val Lys
    50                  55                  60

Asp Phe Leu Ala Ala Asn Pro Ser Val Lys Arg Glu Asp Ile Phe Ile
65                  70                  75                  80

Cys Thr Lys Val Trp Asn His Leu His Arg Pro Glu Asp Val Gln Trp
                85                  90                  95

Ser Ile Glu Ser Ser Leu Lys Lys Leu Lys Val Asp Tyr Ile Asp Leu
            100                 105                 110

Phe Leu Val His Trp Pro Ile Ala Ala Glu Lys Glu Thr Gln Glu Lys
        115                 120                 125

Pro Lys Ile Gly Pro Asp Gly Lys Tyr Val Ile Leu Lys Asp Leu Thr
    130                 135                 140

Glu Asn Pro Glu Pro Thr Trp Arg Ala Met Glu Lys Ile Tyr Lys Glu
145                 150                 155                 160

Lys Lys Ala Lys Ala Ile Gly Val Ser Asn Trp Thr Ile Glu Gly Leu
                165                 170                 175

Glu Lys Leu Leu Lys Tyr Ala Glu Val Lys Pro His Val Asn Gln Ile
            180                 185                 190

Glu Ile His Pro Phe Leu Pro Asn Gln Glu Leu Val Asp Tyr Cys Phe
        195                 200                 205

Lys His Asp Ile Leu Pro Glu Ala Tyr Ser Pro Leu Gly Ser Gln Asn
    210                 215                 220

Gln Val Pro Thr Thr Gly Glu Lys Val Ser Glu Asn Gln Thr Leu Asn
225                 230                 235                 240

Glu Ile Ala Lys Lys Gly Gly Tyr Thr Leu Ala Gln Val Leu Ile Ala
                245                 250                 255

Trp Gly Leu Arg Arg Gly Tyr Val Val Leu Pro Lys Ser Ser Asn Pro
            260                 265                 270

Ala Arg Ile Glu Ser Asn Phe Lys Ser Ile Gln Leu Thr Asp Glu Glu
        275                 280                 285

Tyr Glu Ala Val Asn Lys Val Ala Glu Gly Arg His Phe Arg Phe Val
    290                 295                 300

Asn Met Lys Asp Thr Phe Gly Tyr Asp Val Trp Pro Glu Glu Thr Ala
305                 310                 315                 320

Lys Asn Leu Ser Ala
                325

<210>  5
<211>  906
<212>  DNA
<213>  Aspergillus terreus

<400>  5
atggattcca agatccagac caacgtcccc ctgcccaagg cccccctcat ccagaaggct      60

cgcggcaagc gcaccaaggg catccccgcc ctcgtcgctg gcgcttgtgc cggtgccgtc     120

gagatctcca tcacctaccc cttcgagtcc gccaagactc gtgctcagct caagcgccgc     180

aaccacgatg tcgccgccat caagcccggc atccgcggct ggtacgctgg ctacggcgct     240

actctcgtcg gcaccaccgt caaggcctcc gtccagttcg cctccttcaa catctaccgc     300

tccgccctct ccggccccaa cggtgagctg tccaccggcg cctccgtcct cgctggcttc     360

ggcgctggcg tcaccgaggc cgtcctcgcc gtcacacctg ccgaagccat caagaccaag     420

atcatcgatg cccgcaaggt cggcaacgcc gagctgagca ccaccttcgg cgctatcgcc     480

ggcatcctcc gtgatcgtgg ccctctcggc ttcttctccg ccgtgggccc caccatcctc     540

cgccagtcct ccaacgccgc cgtcaagttc accgtctaca acgagctgat cggcctcgcc     600

cgcaagtact ccaagaacgg cgaggatgtc caccccctcg cctccaccct ggtcggctcc     660

gtcaccggcg tctgctgcgc ctggtccacc cagcccctcg atgtcatcaa gacccgcatg     720

cagtccctcc aggcccgcca gctctacggc aacaccttca actgcgtcaa gaccctcctg     780

cgctccgagg gcatcggcgt gttctggtcc ggcgtctggt tccgcaccgg tcgcctctcc     840

ctcacctctg ccatcatgtt ccccgtttac gagaaggtct acaagttcct cacccagccc 900

aactga 906

<210> 6
<211> 301
<212> PRT
<213> Aspergillus terreus

<400> 6

Met Asp Ser Lys Ile Gln Thr Asn Val Pro Leu Pro Lys Ala Pro Leu
1               5                   10                  15

Ile Gln Lys Ala Arg Gly Lys Arg Thr Lys Gly Ile Pro Ala Leu Val
            20                  25                  30

Ala Gly Ala Cys Ala Gly Ala Val Glu Ile Ser Ile Thr Tyr Pro Phe
        35                  40                  45

Glu Ser Ala Lys Thr Arg Ala Gln Leu Lys Arg Arg Asn His Asp Val
    50                  55                  60

Ala Ala Ile Lys Pro Gly Ile Arg Gly Trp Tyr Ala Gly Tyr Gly Ala
65                  70                  75                  80

Thr Leu Val Gly Thr Thr Val Lys Ala Ser Val Gln Phe Ala Ser Phe
                85                  90                  95

Asn Ile Tyr Arg Ser Ala Leu Ser Gly Pro Asn Gly Glu Leu Ser Thr
            100                 105                 110

Gly Ala Ser Val Leu Ala Gly Phe Gly Ala Gly Val Thr Glu Ala Val
        115                 120                 125

Leu Ala Val Thr Pro Ala Glu Ala Ile Lys Thr Lys Ile Ile Asp Ala
    130                 135                 140

Arg Lys Val Gly Asn Ala Glu Leu Ser Thr Thr Phe Gly Ala Ile Ala
145                 150                 155                 160

Gly Ile Leu Arg Asp Arg Gly Pro Leu Gly Phe Phe Ser Ala Val Gly
                165                 170                 175

Pro Thr Ile Leu Arg Gln Ser Ser Asn Ala Ala Val Lys Phe Thr Val
            180                 185                 190

Tyr Asn Glu Leu Ile Gly Leu Ala Arg Lys Tyr Ser Lys Asn Gly Glu
        195                 200                 205

Asp Val His Pro Leu Ala Ser Thr Leu Val Gly Ser Val Thr Gly Val
    210                 215                 220

Cys Cys Ala Trp Ser Thr Gln Pro Leu Asp Val Ile Lys Thr Arg Met
225                 230                 235                 240

Gln Ser Leu Gln Ala Arg Gln Leu Tyr Gly Asn Thr Phe Asn Cys Val
                245                 250                 255

Lys Thr Leu Leu Arg Ser Glu Gly Ile Gly Val Phe Trp Ser Gly Val
            260                 265                 270

Trp Phe Arg Thr Gly Arg Leu Ser Leu Thr Ser Ala Ile Met Phe Pro
        275                 280                 285

Val Tyr Glu Lys Val Tyr Lys Phe Leu Thr Gln Pro Asn
    290                 295                 300

<210>  7
<211>  1470
<212>  DNA
<213>  Aspergillus terreus

<400>  7
atgaccaagc aatctgcgga cagcaacgca aagtcaggag ttacggccga aatatgccat      60

tgggcatcca acctggccac tgacgacatc ccttcggacg tattagaaag agcgaaatac     120

ctgattctcg atggtattgc atgtgcctgg gttggtgcaa gagtgccttg gtcagagaag     180

tatgtgcagg caacaatgag ctttgagccg ccaggagcct gcagggtgat tggatatggg     240

cagaaactgg ggcctgttgc agcagccatg accaattccg ctttcataca ggccacagag     300

cttgacgact accacagcga agccccccta cactctgcaa gcatcgtcct ccctgcggtc     360

tttgcagcaa gtgaggtctt agccgagcaa ggcaaaacaa tttctggtat agatgtcatt     420

ctagccgcca ttgtggggtt tgaatctggc ccgcggatcg gcaaagcaat ttacggatcg     480

gacctcttga acaacggctg gcattgtgga gccgtgtatg gtgctccagc tggtgcgctg     540

gccacaggaa agctcctcgg tctaactcca gactccatgg aagatgctct cggaatcgcg     600

tgcacgcaag cctgtggttt aatgtcggcg caatacggag gcatggtcaa gcgcgtgcaa     660

catggattcg cagcgcgtaa tggtcttctt gggggactgt tggcctatgg tgggtacgag     720

gccatgaagg gtgtcctgga gagatcttat ggcggtttcc tcaaaatgtt caccaagggc     780

aatggcagag agcctcccta caaagaggag gaagtggtgg ccggtctcgg ttcattctgg     840

cataccttta ctattcgcat caagctctat gcctgctgcg gacttgtcca tggtccagtc     900

gaagctatcg aaaagcttca gaggagatac cccgagctct tgaatagagc caacctcagc     960

aacattcgcc atgtttatgt acagctttca acagcctcga acagtcactg tggatggata 1020

ccagaggaga ggcccatcag ttcaatcgca gggcagatga gtgtcgcata catcctcgcc 1080

gtccagctgg tcgaccagca atgtcttctg gctcagtttt ctgagtttga tgacaacttg 1140

gagagaccag aagtgtggga tctggccagg aaggttactc catctcatag cgaagagttt 1200

gatcaagacg gcaactgtct cagtgcgggt cgcgtgagga ttgagttcaa cgatggctct 1260

tctgttacgg aaactgtcga gaagcctctt ggagtcaaag agcccatgcc aaacgaacgg 1320

attctccaca aataccgaac ccttgcgggt agcgtgacgg acgaatcccg ggtgaaagag 1380

attgaggatc ttgtcctcag cctggacagg ctcaccgaca ttaccccatt gctggagctg 1440

cttaattgtc ccgtgaaatc gccactggta 1470

<210> 8
<211> 490
<212> PRT
<213> Aspergillus terreus

<400> 8

Met Thr Lys Gln Ser Ala Asp Ser Asn Ala Lys Ser Gly Val Thr Ala
1               5                   10                  15

Glu Ile Cys His Trp Ala Ser Asn Leu Ala Thr Asp Asp Ile Pro Ser
            20                  25                  30

Asp Val Leu Glu Arg Ala Lys Tyr Leu Ile Leu Asp Gly Ile Ala Cys
        35                  40                  45

Ala Trp Val Gly Ala Arg Val Pro Trp Ser Glu Lys Tyr Val Gln Ala
    50                  55                  60

Thr Met Ser Phe Glu Pro Pro Gly Ala Cys Arg Val Ile Gly Tyr Gly
65                  70                  75                  80

Gln Lys Leu Gly Pro Val Ala Ala Ala Met Thr Asn Ser Ala Phe Ile
                85                  90                  95

Gln Ala Thr Glu Leu Asp Asp Tyr His Ser Glu Ala Pro Leu His Ser
            100                 105                 110

Ala Ser Ile Val Leu Pro Ala Val Phe Ala Ala Ser Glu Val Leu Ala
        115                 120                 125

Glu Gln Gly Lys Thr Ile Ser Gly Ile Asp Val Ile Leu Ala Ala Ile
    130                 135                 140

Val Gly Phe Glu Ser Gly Pro Arg Ile Gly Lys Ala Ile Tyr Gly Ser
145                 150                 155                 160

Asp Leu Leu Asn Asn Gly Trp His Cys Gly Ala Val Tyr Gly Ala Pro
                165                 170                 175

Ala Gly Ala Leu Ala Thr Gly Lys Leu Leu Gly Leu Thr Pro Asp Ser
            180                 185                 190

Met Glu Asp Ala Leu Gly Ile Ala Cys Thr Gln Ala Cys Gly Leu Met
        195                 200                 205

Ser Ala Gln Tyr Gly Gly Met Val Lys Arg Val Gln His Gly Phe Ala
    210                 215                 220

Ala Arg Asn Gly Leu Leu Gly Gly Leu Leu Ala Tyr Gly Gly Tyr Glu
225                 230                 235                 240

Ala Met Lys Gly Val Leu Glu Arg Ser Tyr Gly Gly Phe Leu Lys Met
                245                 250                 255

Phe Thr Lys Gly Asn Gly Arg Glu Pro Pro Tyr Lys Glu Glu Glu Val
            260                 265                 270

Val Ala Gly Leu Gly Ser Phe Trp His Thr Phe Thr Ile Arg Ile Lys
        275                 280                 285

Leu Tyr Ala Cys Cys Gly Leu Val His Gly Pro Val Glu Ala Ile Glu
    290                 295                 300

Lys Leu Gln Arg Arg Tyr Pro Glu Leu Leu Asn Arg Ala Asn Leu Ser
305                 310                 315                 320

Asn Ile Arg His Val Tyr Val Gln Leu Ser Thr Ala Ser Asn Ser His
                325                 330                 335

Cys Gly Trp Ile Pro Glu Glu Arg Pro Ile Ser Ser Ile Ala Gly Gln
            340                 345                 350

Met Ser Val Ala Tyr Ile Leu Ala Val Gln Leu Val Asp Gln Gln Cys
        355                 360                 365

Leu Leu Ala Gln Phe Ser Glu Phe Asp Asp Asn Leu Glu Arg Pro Glu
    370                 375                 380

Val Trp Asp Leu Ala Arg Lys Val Thr Pro Ser His Ser Glu Glu Phe
385                 390                 395                 400

Asp Gln Asp Gly Asn Cys Leu Ser Ala Gly Arg Val Arg Ile Glu Phe
                405                 410                 415

Asn Asp Gly Ser Ser Val Thr Glu Thr Val Glu Lys Pro Leu Gly Val
            420                 425                 430

Lys Glu Pro Met Pro Asn Glu Arg Ile Leu His Lys Tyr Arg Thr Leu
        435                 440                 445

Ala Gly Ser Val Thr Asp Glu Ser Arg Val Lys Glu Ile Glu Asp Leu
    450                 455                 460

Val Leu Ser Leu Asp Arg Leu Thr Asp Ile Thr Pro Leu Leu Glu Leu
465                 470                 475                 480

Leu Asn Cys Pro Val Lys Ser Pro Leu Val
                485                 490

<210>  9
<211>  1395
<212>  DNA
<213>  Aspergillus terreus

<400>  9
atgggccacg gcgataccga gtcccccaac cccaccacca ccaccgaggg ctccggccag      60

aacgagcccg agaagaaggg ccgcgatatc cccctgtggc gcaagtgcgt catcaccttc     120

gtcgtgtcct ggatgaccct cgtcgtcacc ttctcctcca cctgcctgct ccccgctgcc     180

cccgagatcg ccaacgagtt cgatatgacc gtcgaaacca tcaacatctc caacgctggc     240

gtcctcgtcg ctatgggcta ctcctccctc atctggggcc ctatgaacaa gctcgtcggt     300

cgccgcacct cctacaacct cgccatctcc atgctctgcg cctgctccgc cggcaccgcc     360

gctgccatca acgaggaaat gttcatcgcc ttccgcgtcc tgtctggcct caccggcacc     420

agcttcatgg tgtccggcca gaccgtcctc gccgatatct tcgagcccgt ctaccgcggc     480

accgccgtcg gtttcttcat ggccggcacc ctcagcggcc ctgccatcgg cccttgcgtc     540

ggcggcgtca tcgtgacctt caccagctgg cgcgtcatct tctggctcca gctcggcatg     600

tccggcctcg gcctcgtcct ctccctcctg ttcttcccga agatcgaggg caactccgag     660

aaggtgtcca ccgccttcaa gcccaccacc ctcgtcacca tcatctccaa gttctccccc     720

accgatgtcc tcaagcagtg ggtctacccc aacgtgttcc tcgccgatct ctgctgcggc     780

ctcctcgcca tcacccagta ctccatcctc acctccgctc gcgccatctt caactcccgc     840

ttccacctca ccaccgccct cgtgtccggc ctcttctacc tcgcccctgg cgccggtttc     900

ctcatcggct ccctcgtcgg cggcaagctc tccgatcgca ccgtccgccg ctacatcgtc     960

aagcgcggct tccgcctccc ccaggatcgc ctccactccg gcctcatcac cctcttcgcc 1020

gtcctccctg ccggcaccct gatctacggc tggaccctcc aagaggataa gggcgatatg 1080

gtcgtcccca tcattgccgc tttcttcgcc ggctggggtc tgatgggctc cttcaactgc 1140

ctcaacacct acgtcgccga ggccctccct cgcaaccgct ctgccgtgat cgccggcaag 1200

tacatgatcc agtacacctt ctccgccggc tcctccgctc tcgtcgtccc cgtgatcgat 1260

gccctcggcg tcggcgaaac cttcaccctc tgcgtcgtcg cctccaccat cgccggcctg 1320

atcaccgccg ctatcgcccg ctggggcatc aacatgcagc gctgggccga acgcgctttc 1380

aacatgccca cccag 1395

<210> 10
<211> 465
<212> PRT
<213> Aspergillus terreus

<400> 10

Met Gly His Gly Asp Thr Glu Ser Pro Asn Pro Thr Thr Thr Thr Glu
1               5                   10                  15

Gly Ser Gly Gln Asn Glu Pro Glu Lys Lys Gly Arg Asp Ile Pro Leu
            20                  25                  30

Trp Arg Lys Cys Val Ile Thr Phe Val Val Ser Trp Met Thr Leu Val
        35                  40                  45

Val Thr Phe Ser Ser Thr Cys Leu Leu Pro Ala Ala Pro Glu Ile Ala
    50                  55                  60

Asn Glu Phe Asp Met Thr Val Glu Thr Ile Asn Ile Ser Asn Ala Gly
65                  70                  75                  80

Val Leu Val Ala Met Gly Tyr Ser Ser Leu Ile Trp Gly Pro Met Asn
                85                  90                  95

Lys Leu Val Gly Arg Arg Thr Ser Tyr Asn Leu Ala Ile Ser Met Leu
            100                 105                 110

Cys Ala Cys Ser Ala Gly Thr Ala Ala Ala Ile Asn Glu Glu Met Phe
        115                 120                 125

Ile Ala Phe Arg Val Leu Ser Gly Leu Thr Gly Thr Ser Phe Met Val
    130                 135                 140

Ser Gly Gln Thr Val Leu Ala Asp Ile Phe Glu Pro Val Tyr Arg Gly
145                 150                 155                 160

Thr Ala Val Gly Phe Phe Met Ala Gly Thr Leu Ser Gly Pro Ala Ile
                165                 170                 175

Gly Pro Cys Val Gly Gly Val Ile Val Thr Phe Thr Ser Trp Arg Val
            180                 185                 190

Ile Phe Trp Leu Gln Leu Gly Met Ser Gly Leu Gly Leu Val Leu Ser
        195                 200                 205

Leu Leu Phe Phe Pro Lys Ile Glu Gly Asn Ser Glu Lys Val Ser Thr
    210                 215                 220

Ala Phe Lys Pro Thr Thr Leu Val Thr Ile Ile Ser Lys Phe Ser Pro
225                 230                 235                 240

Thr Asp Val Leu Lys Gln Trp Val Tyr Pro Asn Val Phe Leu Ala Asp
                245                 250                 255

Leu Cys Cys Gly Leu Leu Ala Ile Thr Gln Tyr Ser Ile Leu Thr Ser
            260                 265                 270

Ala Arg Ala Ile Phe Asn Ser Arg Phe His Leu Thr Thr Ala Leu Val
        275                 280                 285

Ser Gly Leu Phe Tyr Leu Ala Pro Gly Ala Gly Phe Leu Ile Gly Ser
    290                 295                 300

Leu Val Gly Gly Lys Leu Ser Asp Arg Thr Val Arg Arg Tyr Ile Val
305                 310                 315                 320

Lys Arg Gly Phe Arg Leu Pro Gln Asp Arg Leu His Ser Gly Leu Ile
                325                 330                 335

Thr Leu Phe Ala Val Leu Pro Ala Gly Thr Leu Ile Tyr Gly Trp Thr
            340                 345                 350

Leu Gln Glu Asp Lys Gly Asp Met Val Val Pro Ile Ile Ala Ala Phe
        355                 360                 365

Phe Ala Gly Trp Gly Leu Met Gly Ser Phe Asn Cys Leu Asn Thr Tyr
    370                 375                 380

Val Ala Glu Ala Leu Pro Arg Asn Arg Ser Ala Val Ile Ala Gly Lys
385                 390                 395                 400

Tyr Met Ile Gln Tyr Thr Phe Ser Ala Gly Ser Ser Ala Leu Val Val
                405                 410                 415

Pro Val Ile Asp Ala Leu Gly Val Gly Glu Thr Phe Thr Leu Cys Val
            420                 425                 430

Val Ala Ser Thr Ile Ala Gly Leu Ile Thr Ala Ala Ile Ala Arg Trp
        435                 440                 445

Gly Ile Asn Met Gln Arg Trp Ala Glu Arg Ala Phe Asn Met Pro Thr
    450                 455                 460

Gln
465

<210>  11
<211>  476
<212>  PRT
<213>  Aspergillus flavus

<400>  11

Met Ser Cys Gln Lys Val Leu Ser Met Gln Leu Lys Ala Thr Ser Pro
1               5                   10                  15

Leu Pro Ile His Arg Arg Ile Val Leu Leu Lys Val Leu Ser Ile Gln
            20                  25                  30

Ala Leu Val Tyr Met Ile Val Gln Gly Ile Ser Pro Thr Phe Trp Gly
        35                  40                  45

Ser Ile Ser Asp Ala Thr Gly Arg Arg Pro Val Phe Ile Gly Thr Phe
    50                  55                  60

Ile Val Tyr Met Ile Ala Asn Val Ala Leu Ala Val Ser Thr Lys Tyr
65                  70                  75                  80

Gly Glu Leu Met Ala Phe Arg Ala Leu Gln Ala Ala Gly Ser Ala Ala
                85                  90                  95

Thr Ile Ser Ile Gly Ala Gly Val Ile Gly Asp Ile Thr Thr Ser Ala
            100                 105                 110

Glu Arg Gly Ser Leu Val Gly Ile Phe Gly Gly Gly Lys Ile Leu Val
        115                 120                 125

Arg Pro Ile Leu Arg Leu Tyr Trp Ala Leu Gly Gly His His Glu Leu
    130                 135                 140

Arg Met Leu Gly Gln Gly Ile Gly Pro Val Met Gly Gly Ile Leu Thr
145                 150                 155                 160

Gln Tyr Leu Gly Phe Arg Ser Ile Phe Trp Phe Leu Thr Ile Cys Ala
                165                 170                 175

Ala Val Ser Leu Leu Ser Ile Leu Ile Phe Leu Pro Glu Thr Leu Arg
            180                 185                 190

His Ile Ala Gly Asn Gly Thr Val Arg Leu Arg Gly Ile His Lys Pro
        195                 200                 205

Phe Leu Tyr Val Val Ile Gly Gln Lys Gly Ala Ile Thr Gly Ala Asp
    210                 215                 220

Pro Gly Gln Lys Lys Pro Glu Leu Thr Trp Arg Ala Ile Leu Ala Pro
225                 230                 235                 240

Leu Thr Phe Leu Ala Glu Lys Asp Ile Phe Val Thr Leu Leu Phe Gly
                245                 250                 255

Ser Ile Val Tyr Ala Val Trp Ser Met Val Thr Ser Ser Thr Thr Asp
            260                 265                 270

Leu Phe Gln Asp Val Tyr His Leu Thr Ser Leu Glu Val Gly Leu Thr
        275                 280                 285

Phe Leu Gly Asn Gly Phe Gly Cys Ile Ser Gly Ser Tyr Leu Val Gly
    290                 295                 300

Tyr Leu Met Asp Tyr Asn His Lys Leu Thr Glu Arg Glu Tyr Cys Asp
305                 310                 315                 320

Lys Tyr Gly Tyr Pro Ser Gly Thr Arg Val Asn Leu Lys Ser His Ser
                325                 330                 335

Asp Phe Pro Ile Glu Val Ala Arg Met Arg His Thr Trp Trp Ile Val
            340                 345                 350

Gly Leu Phe Ile Val Thr Thr Ala Val Tyr Gly Val Ser Leu Arg Thr
        355                 360                 365

His Ile Ala Val Pro Ile Ile Leu Gln Tyr Leu Ile Ala Leu Cys Ser
    370                 375                 380

Thr Gly Ile Phe Thr Ile Asn Ser Ala Leu Val Ile Asp Leu Tyr Pro
385                 390                 395                 400

Gly Ala Ser Ala Ser Ala Thr Ala Val Asn Asn Leu Ile Arg Cys Leu

405                 410                 415

Val Gly Ala Ala Gly Val Ala Ala Met Gln Pro Met Leu Asp Val Leu
            420                 425                 430

Thr Pro Asp Tyr Val Phe Leu Leu Leu Ala Gly Ile Thr Leu Ile Met
        435                 440                 445

Ala Pro Leu Leu Trp Met Glu Ser Arg Phe Gly Ala Ser Trp Arg His
    450                 455                 460

Glu Arg Glu Met Arg Leu Lys Asp Lys Gly Cys Thr
465                 470                 475

<210>  12
<211>  515
<212>  PRT
<213>  Aspergillus oryzae

<400>  12

Met Ala Thr Ala Arg Ser Asp Thr Asp Lys Ala Pro Ile Val Ser Phe
1               5                   10                  15

Val Glu Glu Asp Glu Arg Gln Pro Ser Asp Ser Gln Leu Glu Asp Gln
            20                  25                  30

Val Thr Gln Ser Lys Ser His His Ile Phe Ser Arg Arg Lys Lys Leu
        35                  40                  45

Gln Met Val Cys Ile Val Ser Met Ala Ala Ile Phe Ser Pro Leu Ser
    50                  55                  60

Ser Asn Ile Tyr Phe Pro Ala Leu Gly Glu Val Ser Arg Val Ser Ala
65                  70                  75                  80

Thr Lys Thr Lys His Ser Leu Cys Asp Leu Ser Arg Asp Val Tyr Met
                85                  90                  95

Ile Val Gln Gly Ile Ser Pro Thr Phe Trp Gly Ser Ile Ser Asp Ala
            100                 105                 110

Thr Gly Arg Arg Pro Val Phe Ile Gly Thr Phe Ile Val Tyr Met Ile
        115                 120                 125

Ala Asn Val Ala Leu Ala Val Ser Thr Lys Tyr Gly Glu Leu Met Ala
    130                 135                 140

Phe Arg Ala Leu Gln Ala Ala Gly Ser Ala Ala Thr Ile Ser Ile Gly

145                 150                 155                 160

Ala Gly Val Ile Gly Asp Ile Thr Thr Ser Ala Glu Arg Gly Ser Leu
                165                 170                 175

Val Gly Ile Phe Gly Gly Val Arg Met Leu Gly Gln Gly Ile Gly Pro
            180                 185                 190

Val Met Gly Gly Ile Leu Thr Gln Tyr Leu Gly Phe Arg Ser Ile Phe
        195                 200                 205

Trp Phe Leu Thr Ile Cys Ala Ala Val Ser Leu Leu Ser Ile Leu Ile
    210                 215                 220

Phe Leu Pro Glu Thr Leu Arg His Ile Ala Gly Asn Gly Thr Val Arg
225                 230                 235                 240

Leu Arg Gly Ile His Lys Pro Phe Leu Tyr Val Val Ile Gly Gln Lys
                245                 250                 255

Gly Ala Ile Thr Gly Ala Asp Pro Gly Gln Lys Lys Pro Glu Leu Thr
            260                 265                 270

Trp Arg Ala Ile Leu Ala Pro Leu Thr Phe Leu Ala Glu Lys Asp Ile
        275                 280                 285

Phe Val Thr Leu Leu Phe Gly Ser Ile Val Tyr Ala Val Trp Ser Met
    290                 295                 300

Val Thr Ser Ser Thr Thr Asp Leu Phe Gln Asp Val Tyr His Leu Thr
305                 310                 315                 320

Ser Leu Glu Val Gly Leu Thr Phe Leu Gly Asn Gly Phe Gly Cys Ile
                325                 330                 335

Ser Gly Ser Tyr Leu Val Gly Tyr Leu Met Asp Tyr Asn His Lys Leu
            340                 345                 350

Thr Glu Arg Glu Tyr Cys Asp Lys Tyr Gly Tyr Pro Ser Gly Thr Arg
        355                 360                 365

Val Asn Leu Lys Ser His Ser Asp Phe Pro Ile Glu Val Ala Arg Met
    370                 375                 380

Arg His Thr Trp Trp Ile Val Gly Leu Phe Ile Val Thr Thr Ala Val
385                 390                 395                 400

Tyr Gly Val Ser Leu Arg Thr His Ile Ala Val Pro Ile Ile Leu Gln
                405                 410                 415

Tyr Leu Ile Ala Leu Cys Ser Thr Gly Ile Phe Thr Ile Asn Ser Ala
            420                 425                 430

Leu Val Ile Asp Leu Tyr Pro Gly Ala Ser Ala Ser Ala Thr Ala Val
        435                 440                 445

Asn Asn Leu Ile Arg Cys Leu Val Gly Ala Ala Gly Val Ala Ala Met
    450                 455                 460

Gln Pro Met Leu Asp Val Leu Thr Pro Asp Tyr Val Phe Leu Leu Leu
465                 470                 475                 480

Ala Gly Ile Thr Leu Ile Met Ala Pro Leu Leu Trp Met Glu Ser Arg
                485                 490                 495

Phe Gly Ala Ser Trp Arg His Glu Arg Glu Met Arg Leu Lys Asp Lys
            500                 505                 510

Gly Cys Thr
        515

<210> 13
<211> 211
<212> PRT
<213> Aspergillus clavatus

<400> 13

Met Val Ser Val Gly Ser Asp Thr Pro Ser Asp Thr Arg Lys Thr Ala
1               5                   10                  15

Ile Glu Pro Val Val Thr Ser Arg Glu Ile Ser Ser Ser Leu Val Ser
            20                  25                  30

Pro Gln Leu Glu Gly Gln Tyr Pro Glu Pro Pro Ala Tyr His Val Phe
        35                  40                  45

Ser Arg Ser Arg Lys Leu Gln Met Val Cys Ile Val Ser Leu Ala Ala
    50                  55                  60

Ile Phe Ser Pro Leu Ser Ser Asn Ile Tyr Phe Pro Ala Leu Gly Asp
65                  70                  75                  80

Val Ser Arg Ala Leu Asp Ile Ser Met Thr Leu Ala Thr Leu Thr Ile
                85                  90                  95

Thr Val Tyr Met Ile Val Gln Gly Thr Ala Pro Thr Leu Trp Gly Ser
            100                 105                 110

Ile Ser Asp Val Thr Gly Arg Arg Pro Val Phe Ile Gly Thr Phe Val
        115                 120                 125

Val Tyr Met Ile Ala Asn Val Ala Leu Ala Met Ser Lys Asn Tyr Gly
    130                 135                 140

Glu Leu Met Ala Phe Arg Ala Leu Gln Ala Ala Gly Ser Ala Ala Thr
145                 150                 155                 160

Ile Ser Asn Gly Phe Gly Cys Met Leu Gly Ser Tyr Leu Ile Gly Tyr
                165                 170                 175

Leu Met Asp Tyr Asn His Arg Leu Thr Glu Arg Glu Tyr Cys Glu Lys
            180                 185                 190

Gln Gly Tyr Pro Ala Gly Thr Arg Val Asn Leu Lys Ser His Ser Asp
        195                 200                 205

Ile Ser Asn
    210

<210>  14
<211>  512
<212>  PRT
<213>  Aspergillus wentii

<400>  14

Met Asp Ile Ser Lys Glu Lys Ala Pro Glu Ala Thr Val Thr Ser Leu
1               5                   10                  15

Pro Gly Ser Arg Gln Thr Ser Pro Pro Glu Ser Ile Lys Leu Glu Gly
            20                  25                  30

Gln Pro Glu Ile Thr Pro Pro Pro His His Val Phe Ser Arg Gly Gln
        35                  40                  45

Lys Leu Gln Met Val Tyr Ile Val Ser Leu Ala Ala Ile Phe Ser Pro
    50                  55                  60

Leu Ser Ser Asn Ile Tyr Phe Pro Ala Leu Gly Asp Ile Ser Lys Ser
65                  70                  75                  80

Leu Asn Thr Ser Met Ser Leu Val Thr Leu Thr Val Thr Val Tyr Met
                85                  90                  95

Ile Val Gln Gly Leu Ala Pro Thr Phe Trp Gly Ser Met Ser Asp Ala
            100                 105                 110

Thr Gly Arg Arg Pro Val Phe Ile Gly Thr Phe Ala Val Tyr Leu Ile
        115                 120                 125

Ala Asn Ile Val Leu Ala Val Ser Thr Asn Tyr Pro Glu Leu Met Val
    130                 135                 140

Phe Arg Ala Leu Gln Ala Ala Gly Ser Ala Ala Thr Ile Ser Ile Gly
145                 150                 155                 160

Ala Gly Val Ile Gly Asp Met Thr Thr Ser Ser Glu Arg Gly Ser Leu
                165                 170                 175

Val Gly Ile Phe Gly Gly Val Arg Met Leu Gly Gln Gly Val Gly Pro
            180                 185                 190

Val Phe Gly Gly Ile Leu Ser Glu Tyr Leu Gly Phe Arg Ser Ile Phe
        195                 200                 205

Trp Phe Leu Thr Ile Ala Gly Ala Ile Ser Leu Leu Ser Ile Leu Val
    210                 215                 220

Phe Leu Pro Glu Thr Leu Arg Pro Ile Ala Gly Asn Gly Ser Val Pro
225                 230                 235                 240

Leu Thr Gly Phe His Lys Pro Phe Ile Tyr Tyr Ile Phe Pro Gln Lys
                245                 250                 255

His Ala Gln Glu Gly Ala Thr Pro Gly Val Glu Lys Thr Lys Val Thr
            260                 265                 270

Trp Lys Ala Val Phe Ser Pro Leu Thr Phe Leu Phe Glu Lys Asp Val
        275                 280                 285

Phe Ile Thr Leu Phe Phe Gly Ser Ile Val Tyr Thr Phe Trp Ser Met
    290                 295                 300

Val Thr Ser Ser Thr Thr Ser Leu Phe Gln Glu Val Tyr Gly Leu Ser
305                 310                 315                 320

Thr Leu Gln Val Gly Leu Thr Phe Leu Gly Asn Gly Phe Gly Cys Met
                325                 330                 335

Ser Gly Ser Tyr Ser Val Gly Tyr Leu Met Asp Tyr Asn His Arg Met
            340                 345                 350

Thr Glu Arg Glu Tyr Cys Glu Lys Arg Gly Tyr Pro Ser Gly Thr Arg
        355                 360                 365

Val Asn Leu Lys Ser His Ala Asp Phe Pro Ile Glu Val Ala Arg Met
    370                 375                 380

Arg Asn Thr Trp Trp Val Ile Ala Leu Phe Val Ile Thr Thr Ala Leu
385                 390                 395                 400

Tyr Gly Val Ser Leu Arg Thr His Leu Ala Val Pro Ile Ile Leu Gln
                405                 410                 415

Tyr Phe Ile Ala Phe Cys Ala Thr Gly Ile Phe Thr Ile Asn Ser Ala
            420                 425                 430

Phe Val Ile Asp Leu Tyr Pro Gly Ala Ser Ala Ser Ala Thr Ala Val
        435                 440                 445

Asn Asn Leu Ile Arg Cys Leu Ile Gly Ala Ala Gly Val Ala Val Val
    450                 455                 460

Gln Pro Ile Ile Asp Ala Leu Thr Ala Glu Tyr Thr Phe Ile Met Leu
465                 470                 475                 480

Ala Gly Ile Thr Val Gly Met Thr Pro Leu Leu Trp Ile Glu Ser Lys
                485                 490                 495

Tyr Gly Pro Gly Trp Arg His Ala Arg Glu Glu Arg Leu Ala Arg Lys
            500                 505                 510

<210>  15
<211>  522
<212>  PRT
<213>  Aspergillus aculeatus

<400>  15

Met Ser Leu Ser Arg Glu Glu Thr Ala Lys Val Pro Glu Pro Gln Leu
1               5                   10                  15

Ser Ser Val Ala Ser Phe Thr Asp Gln Asp Ser Glu Lys Gly Ser Ile
            20                  25                  30

Ser Arg Asp Pro Ser Thr Phe Glu Gly Gln Ser Ser Glu Pro Pro Pro
        35                  40                  45

His His Ile Phe Thr Arg Ser Arg Lys Leu Gln Met Val Cys Ile Val
    50                  55                  60

Ser Met Ala Ala Ile Phe Ser Pro Leu Ser Ser Asn Ile Tyr Phe Pro
65                  70                  75                  80

Ala Leu Asp Asp Val Ser Lys Ser Leu Asn Val Ser Met Thr Leu Ala
                85                  90                  95

Thr Leu Thr Ile Thr Ile Tyr Met Ile Val Gln Gly Leu Ala Pro Thr
            100                 105                 110

Phe Trp Gly Ser Leu Ser Asp Ala Leu Gly Arg Arg Pro Val Phe Ile
        115                 120                 125

Gly Thr Phe Val Val Tyr Leu Val Ala Asn Ile Ala Leu Ala Glu Ser
    130                 135                 140

Lys Asn Tyr Gly Glu Leu Met Ala Phe Arg Ala Leu Gln Ala Ala Gly
145                 150                 155                 160

Ser Ala Ala Thr Ile Ser Ile Gly Ala Gly Val Ile Gly Asp Ile Thr
                165                 170                 175

Thr Ser Ala Glu Arg Gly Ser Leu Val Gly Ile Phe Gly Gly Val Arg
            180                 185                 190

Met Leu Gly Gln Gly Ile Gly Pro Val Phe Gly Gly Leu Leu Thr Gln
        195                 200                 205

Tyr Leu Gly Tyr Arg Ser Ile Phe Trp Phe Leu Thr Ile Leu Gly Gly
    210                 215                 220

Val Ser Leu Val Ser Ile Leu Val Leu Leu Pro Glu Thr Leu Arg Pro
225                 230                 235                 240

Ile Ala Gly Asn Gly Thr Val Lys Leu Asp Gly Leu His Lys Pro Phe
                245                 250                 255

Leu Tyr His Phe Thr Ser Gln Pro Gly Val Ile Asp Gly Ala Glu Pro
            260                 265                 270

Gly Val Lys Lys Ala Lys Val Thr Trp Arg Thr Val Cys Ala Pro Leu
        275                 280                 285

Lys Phe Leu Ala Glu Lys Asp Val Phe Ile Thr Leu Phe Phe Gly Ser
    290                 295                 300

Ile Val Tyr Thr Val Trp Ser Met Val Thr Ser Ser Thr Thr Asp Leu
305                 310                 315                 320

Phe Ser Glu Val Tyr Gly Leu Ser Ser Leu Glu Val Gly Leu Thr Phe
                325                 330                 335

Leu Gly Asn Gly Phe Gly Cys Met Ser Gly Ser Tyr Leu Val Gly Tyr
            340                 345                 350

Leu Met Asp Tyr Asn His Arg Leu Thr Glu Arg Glu Tyr Cys Glu Lys
        355                 360                 365

His Gly Tyr Pro Ala Gly Thr Arg Val Asn Leu Lys Ser His Ser Asp
    370                 375                 380

Phe Pro Ile Glu Val Ala Arg Met Arg Asn Thr Trp Trp Ile Thr Gly
385                 390                 395                 400

Ile Phe Ile Val Thr Thr Ala Val Tyr Gly Val Ser Leu Arg Thr His
                405                 410                 415

Val Ala Val Pro Ile Ile Leu Gln Tyr Phe Ile Ala Phe Cys Ala Thr
            420                 425                 430

Gly Ile Phe Thr Ile Asn Ser Ala Leu Val Ile Asp Leu Tyr Pro Gly
        435                 440                 445

Ala Ser Ala Ser Ala Thr Ala Val Asn Asn Leu Met Arg Cys Leu Ile
    450                 455                 460

Gly Ala Ala Gly Val Ala Ala Val Gln Pro Ile Leu Glu Ala Leu Gly
465                 470                 475                 480

Ala Asp Tyr Thr Phe Val Leu Leu Ala Gly Ile Thr Leu Val Met Val
                485                 490                 495

Pro Leu Leu Trp Ile Glu Asp Arg Trp Gly Pro Gly Trp Arg His Ala
            500                 505                 510

Arg Glu Arg Arg Leu Lys Ala Gln Met Pro
        515                 520

<210>  16
<211>  519
<212>  PRT
<213>  Aspergillus carbonarius

<400>  16

Met Ser Ser Arg Thr Asp Ile Glu Lys Val Pro Glu Pro Gln Val Thr
1               5                   10                  15

Pro Lys Asp Leu Asp Ser Glu Lys Asp Ala Thr Ser Pro Glu Pro Ser
            20                  25                  30

Val Leu Glu Ser Gln Pro Ser Glu Lys Pro Pro His His Ile Phe Thr
        35                  40                  45

Arg Ser Arg Lys Leu Gln Met Val Gly Ile Val Ser Leu Ala Ala Ile
    50                  55                  60

Phe Ser Pro Leu Ser Ser Asn Ile Tyr Phe Pro Ala Leu Asp Asp Val
65                  70                  75                  80

Ser Gln Ser Leu His Ile Ser Met Ser Leu Ala Thr Leu Thr Ile Thr
                85                  90                  95

Val Tyr Met Ile Val Gln Gly Leu Ala Pro Ser Phe Trp Gly Ser Leu
            100                 105                 110

Ser Asp Ala Thr Gly Arg Arg Pro Val Phe Ile Gly Thr Phe Ile Val
        115                 120                 125

Tyr Leu Ile Ala Asn Ile Ala Leu Gly Glu Ser Lys Asn Tyr Gly Glu
    130                 135                 140

Leu Met Ala Phe Arg Ala Leu Gln Ala Ala Gly Ser Ala Ala Thr Ile
145                 150                 155                 160

Ser Ile Gly Ala Gly Val Ile Gly Asp Ile Thr Thr Ser Glu Glu Arg
                165                 170                 175

Gly Ser Leu Val Gly Ile Phe Gly Gly Val Arg Met Leu Gly Gln Gly
            180                 185                 190

Ile Gly Pro Val Phe Gly Gly Ile Phe Thr Gln Tyr Phe Gly Tyr Arg
        195                 200                 205

Ser Ile Phe Trp Phe Leu Thr Ala Phe Gly Gly Val Ser Leu Leu Ser
    210                 215                 220

Ile Met Val Leu Leu Pro Glu Thr Leu Arg Pro Ile Ala Gly Asn Gly
225                 230                 235                 240

Thr Val Lys Leu Thr Gly Met His Lys Pro Phe Ile Tyr Ser Ile Thr
                245                 250                 255

Gly Gln Lys Gly Val Ile Glu Gly Ala Leu Pro Asp Ala Arg Lys Gly

            260                 265                 270

Lys Val Thr Trp Arg Ala Val Phe Ala Pro Leu Thr Phe Leu Ile Glu
        275                 280                 285

Lys Asp Val Phe Ile Thr Leu Phe Phe Gly Ser Val Val Tyr Thr Val
    290                 295                 300

Trp Ser Met Val Thr Ser Ser Thr Thr Asp Leu Phe Ser Glu Val Tyr
305                 310                 315                 320

Gly Leu Ser Ser Leu Gln Ile Gly Leu Thr Phe Leu Gly Asn Gly Phe
                325                 330                 335

Gly Cys Met Ser Gly Ser Tyr Leu Val Gly Tyr Leu Met Asp Tyr Asn
            340                 345                 350

His Arg Leu Thr Glu Arg Glu Tyr Cys Glu Lys His Gly Tyr Pro Ala
        355                 360                 365

Gly Thr Arg Val Asn Leu Lys Ser His Ser Asp Phe Pro Ile Glu Val
    370                 375                 380

Ala Arg Met Arg Asn Thr Trp Trp Val Ile Ala Ile Phe Val Val Ala
385                 390                 395                 400

Thr Ala Leu Tyr Gly Val Ser Leu Arg Thr His Leu Ala Val Pro Ile
                405                 410                 415

Ile Leu Gln Tyr Phe Ile Ala Phe Cys Ser Thr Gly Leu Phe Thr Ile
            420                 425                 430

Asn Ser Ala Leu Val Ile Asp Leu Tyr Pro Gly Ala Ser Ala Ser Ala
        435                 440                 445

Thr Ala Val Asn Asn Leu Met Arg Cys Leu Leu Gly Ala Gly Gly Val
    450                 455                 460

Ala Val Val Gln Leu Ile Ile Asp Ala Leu Thr Ala Glu Tyr Thr Phe
465                 470                 475                 480

Ile Leu Leu Ala Gly Ile Thr Leu Ala Met Thr Pro Leu Leu Tyr Ile
                485                 490                 495

Glu Asp Arg Trp Gly Pro Ser Trp Arg His Ala Arg Glu Arg Arg Leu
            500                 505                 510

Lys Ala Lys Ala Asn Gly Asn
        515

<210>  17
<211>  524
<212>  PRT
<213>  Aspergillus brasiliensis

<400>  17

Met Ser Ser Thr Thr Ser Ser Ser Arg Ser Asp Leu Glu Lys Val Pro
1               5                   10                  15

Val Thr Gln Val Thr Pro Arg Asp Asn Asp Ser Asp Lys Gly Ser Val
            20                  25                  30

Ser Pro Glu Pro Ser Thr Leu Glu Ala Gln Ser Ser Glu Lys Pro Pro
        35                  40                  45

His His Ile Phe Thr Arg Ser Arg Lys Leu Gln Met Val Cys Ile Val
    50                  55                  60

Ser Leu Ala Ala Ile Phe Ser Pro Leu Ser Ser Asn Ile Tyr Phe Pro
65                  70                  75                  80

Ala Leu Asp Asp Val Ser Lys Ser Leu Asn Ile Ser Met Ser Leu Ala
                85                  90                  95

Thr Leu Thr Ile Thr Val Tyr Met Ile Val Gln Gly Leu Ala Pro Ser
            100                 105                 110

Phe Trp Gly Ser Leu Ser Asp Ala Thr Gly Arg Arg Pro Val Phe Ile
        115                 120                 125

Gly Thr Phe Val Val Tyr Leu Val Ala Asn Ile Ala Leu Ala Val Ser
    130                 135                 140

Lys Asn Tyr Gly Glu Leu Met Ala Phe Arg Ala Leu Gln Ala Ala Gly
145                 150                 155                 160

Ser Ala Ala Thr Ile Ser Ile Gly Ala Gly Val Ile Gly Asp Ile Thr
                165                 170                 175

Asn Ser Glu Glu Arg Gly Ser Leu Val Gly Ile Phe Gly Gly Val Arg
            180                 185                 190

Met Leu Gly Gln Gly Ile Gly Pro Val Phe Gly Gly Ile Phe Thr Gln
        195                 200                 205

Tyr Leu Gly Tyr Arg Ser Ile Phe Trp Phe Leu Thr Ile Phe Gly Gly
    210                 215                 220

Val Ser Leu Leu Ser Ile Leu Val Leu Leu Pro Glu Thr Leu Arg Pro
225                 230                 235                 240

Ile Ala Gly Asn Gly Ser Val Lys Leu His Gly Ile His Lys Pro Phe
                245                 250                 255

Ile Tyr Met Ile Thr Gly Gln Lys Gly Val Ile Glu Gly Ala Gln Pro
            260                 265                 270

Glu Ala Lys Lys Thr Lys Thr Ser Trp Lys Ser Val Phe Ala Pro Leu
        275                 280                 285

Thr Phe Leu Ile Glu Lys Asp Val Phe Ile Thr Leu Phe Phe Gly Ser
    290                 295                 300

Ile Val Tyr Thr Val Trp Ser Met Val Thr Ser Ser Thr Thr Asp Leu
305                 310                 315                 320

Phe Ser Glu Val Tyr Gly Leu Ser Ser Leu Asp Ile Gly Leu Thr Phe
                325                 330                 335

Leu Gly Asn Gly Phe Gly Cys Met Ser Gly Ser Tyr Leu Val Gly Tyr
            340                 345                 350

Leu Met Asp Tyr Asn His Arg Leu Thr Glu Arg Glu Tyr Cys Glu Lys
        355                 360                 365

His Gly Tyr Pro Ala Gly Thr Arg Val Asn Leu Lys Ser His Pro Asp
    370                 375                 380

Phe Pro Ile Glu Val Ala Arg Met Arg Asn Thr Trp Trp Val Ile Ala
385                 390                 395                 400

Ile Phe Ile Val Thr Val Ala Val Tyr Gly Val Ser Leu Arg Thr His
                405                 410                 415

Leu Ala Val Pro Ile Ile Leu Gln Tyr Phe Ile Ala Phe Cys Ser Thr
            420                 425                 430

Gly Leu Phe Thr Ile Asn Ser Ala Leu Val Ile Asp Leu Tyr Pro Gly
        435                 440                 445

Ala Ser Ala Ser Ala Thr Ala Val Asn Asn Leu Met Arg Cys Leu Leu
    450                 455                 460

Gly Ala Gly Gly Val Ala Val Val Gln Pro Ile Leu Asp Ala Leu Asn
465                 470                 475                 480

Pro Asp Tyr Thr Phe Leu Leu Leu Ala Gly Ile Thr Leu Val Met Thr
                485                 490                 495

Pro Leu Leu Tyr Val Glu Asp Arg Trp Gly Pro Gly Trp Arg His Ala
            500                 505                 510

Arg Glu Arg Arg Leu Lys Ala Lys Ala Asn Ser Asn
        515                 520

<210>  18
<211>  524
<212>  PRT
<213>  Aspergillus luchuensis

<400>  18

Met Ser Ser Thr Thr Ser Ser Ser Arg Ser Asp Leu Glu Lys Val Pro
1               5                   10                  15

Val Pro Gln Val Thr Pro Arg Asp Asn Asp Ser Asp Lys Gly Ser Leu
            20                  25                  30

Ser Pro Glu Pro Ser Thr Leu Glu Ala Gln Ser Ser Glu Lys Pro Pro
        35                  40                  45

His His Ile Phe Thr Arg Ser Arg Lys Leu Gln Met Val Cys Ile Val
    50                  55                  60

Ser Leu Ala Ala Ile Phe Ser Pro Leu Ser Ser Asn Ile Tyr Phe Pro
65                  70                  75                  80

Ala Leu Asp Asp Val Ser Lys Ser Leu Asn Ile Ser Met Ser Leu Ala
                85                  90                  95

Thr Leu Thr Ile Thr Val Tyr Met Ile Val Gln Gly Leu Ala Pro Ser
            100                 105                 110

Phe Trp Gly Ser Met Ser Asp Ala Thr Gly Arg Arg Pro Val Phe Ile
        115                 120                 125

Gly Thr Phe Val Val Tyr Leu Val Ala Asn Ile Ala Leu Ala Glu Ser
    130                 135                 140

Lys Asn Tyr Gly Glu Leu Met Ala Phe Arg Ala Leu Gln Ala Ala Gly
145                 150                 155                 160

Ser Ala Ala Thr Ile Ser Ile Gly Ala Gly Val Ile Gly Asp Ile Thr
                165                 170                 175

Asn Ser Glu Glu Arg Gly Ser Leu Val Gly Ile Phe Gly Gly Val Arg
            180                 185                 190

Met Leu Gly Gln Gly Ile Gly Pro Val Phe Gly Gly Ile Phe Thr Gln
        195                 200                 205

Tyr Leu Gly Tyr Arg Ser Ile Phe Trp Phe Leu Thr Ile Ala Gly Gly
    210                 215                 220

Val Ser Leu Leu Ser Ile Leu Val Leu Leu Pro Glu Thr Leu Arg Pro
225                 230                 235                 240

Ile Ala Gly Asn Gly Thr Val Lys Leu Asn Gly Ile His Lys Pro Phe
                245                 250                 255

Ile Tyr Ser Ile Thr Gly Gln Lys Gly Val Val Glu Gly Ala Gln Pro
            260                 265                 270

Glu Ala Lys Lys Lys Gln Ser Ser Trp Lys Ser Val Phe Ala Pro Leu
        275                 280                 285

Thr Phe Leu Val Glu Lys Asp Val Phe Ile Thr Leu Phe Phe Gly Ser
    290                 295                 300

Ile Val Tyr Thr Val Trp Ser Met Val Thr Ser Ser Thr Thr Asp Leu
305                 310                 315                 320

Phe Ser Glu Val Tyr Gly Leu Ser Ser Leu Asp Ile Gly Leu Thr Phe
                325                 330                 335

Leu Gly Asn Gly Phe Gly Cys Met Ser Gly Ser Tyr Leu Val Gly Tyr
            340                 345                 350

Leu Met Asp Tyr Asn His Arg Leu Thr Glu Arg Glu Tyr Cys Glu Lys
        355                 360                 365

His Gly Tyr Pro Ala Gly Thr Arg Val Asn Leu Lys Ser His Pro Asp
    370                 375                 380

Phe Pro Ile Glu Val Ala Arg Met Arg Asn Thr Trp Trp Val Ile Ala
385                 390                 395                 400

Ile Phe Ile Val Thr Val Ala Leu Tyr Gly Val Ser Leu Arg Thr His
                405                 410                 415

Leu Ala Val Pro Ile Ile Leu Gln Tyr Phe Ile Ala Phe Cys Ser Thr
            420                 425                 430

Gly Leu Phe Thr Ile Asn Ser Ala Leu Val Ile Asp Leu Tyr Pro Gly
        435                 440                 445

Ala Ser Ala Ser Ala Thr Ala Val Asn Asn Leu Met Arg Cys Leu Leu
    450                 455                 460

Gly Ala Gly Gly Val Ala Val Val Gln Pro Ile Leu Asp Ala Leu Asn
465                 470                 475                 480

Pro Asp Tyr Thr Phe Leu Leu Leu Ala Gly Ile Thr Leu Val Met Thr
                485                 490                 495

Pro Leu Leu Tyr Val Glu Asp Arg Trp Gly Pro Gly Trp Arg His Ala
            500                 505                 510

Arg Glu Arg Arg Leu Lys Ala Lys Ala Asn Gly Asn
        515                 520

<210>  19
<211>  524
<212>  PRT
<213>  Aspergillus tubingensis

<400>  19

Met Ser Ser Thr Thr Ser Ser Ser Arg Ser Asp Leu Glu Lys Val Pro
1               5                   10                  15

Val Pro Gln Val Thr Pro Arg Asp Asn Asp Ser Asp Lys Gly Ser Leu
            20                  25                  30

Ser Pro Glu Pro Ser Thr Leu Glu Ala Gln Ser Ser Glu Lys Pro Pro
        35                  40                  45

His His Ile Phe Thr Arg Ser Arg Lys Leu Gln Met Val Cys Ile Val
    50                  55                  60

Ser Leu Ala Ala Ile Phe Ser Pro Leu Ser Ser Asn Ile Tyr Phe Pro
65                  70                  75                  80

Ala Leu Asp Asp Val Ser Lys Ser Leu Asn Ile Ser Met Ser Leu Ala
                85                  90                  95

Thr Leu Thr Ile Thr Val Tyr Met Ile Val Gln Gly Leu Ala Pro Ser
            100                 105                 110

Phe Trp Gly Ser Met Ser Asp Ala Thr Gly Arg Arg Pro Val Phe Ile
        115                 120                 125

Gly Thr Phe Val Val Tyr Leu Val Ala Asn Ile Ala Leu Ala Glu Ser
    130                 135                 140

Lys Asn Tyr Gly Glu Leu Met Ala Phe Arg Ala Leu Gln Ala Ala Gly
145                 150                 155                 160

Ser Ala Ala Thr Ile Ser Ile Gly Ala Gly Val Ile Gly Asp Ile Thr
                165                 170                 175

Asn Ser Glu Glu Arg Gly Ser Leu Val Gly Ile Phe Gly Gly Val Arg
            180                 185                 190

Met Leu Gly Gln Gly Ile Gly Pro Val Phe Gly Gly Ile Phe Thr Gln
        195                 200                 205

Tyr Leu Gly Tyr Arg Ser Ile Phe Trp Phe Leu Thr Ile Ala Gly Gly
    210                 215                 220

Val Ser Leu Leu Ser Ile Leu Val Leu Leu Pro Glu Thr Leu Arg Pro
225                 230                 235                 240

Ile Ala Gly Asn Gly Thr Val Lys Leu Asn Gly Ile His Lys Pro Phe
                245                 250                 255

Ile Tyr Ser Ile Thr Gly Gln Lys Gly Val Val Glu Gly Ala Gln Pro
            260                 265                 270

Glu Ala Lys Lys Thr Gln Ser Ser Trp Lys Ser Val Phe Ala Pro Leu
        275                 280                 285

Thr Phe Leu Val Glu Lys Asp Val Phe Ile Thr Leu Phe Phe Gly Ser
    290                 295                 300

Ile Val Tyr Thr Val Trp Ser Met Val Thr Ser Ser Thr Thr Asp Leu
305                 310                 315                 320

Phe Ser Glu Val Tyr Gly Leu Ser Ser Leu Asp Ile Gly Leu Thr Phe
                325                 330                 335

Leu Gly Asn Gly Phe Gly Cys Met Ser Gly Ser Tyr Leu Val Gly Tyr
            340                 345                 350

Leu Met Asp Tyr Asn His Arg Leu Thr Glu Arg Glu Tyr Cys Glu Lys

355                 360                 365

His Gly Tyr Pro Ala Gly Thr Arg Val Asn Leu Lys Ser His Pro Asp
    370                 375                 380

Phe Pro Ile Glu Val Ala Arg Met Arg Asn Thr Trp Trp Val Ile Ala
385                 390                 395                 400

Ile Phe Ile Val Thr Val Ala Leu Tyr Gly Val Ser Leu Arg Thr His
                405                 410                 415

Leu Ala Val Pro Ile Ile Leu Gln Tyr Phe Ile Ala Phe Cys Ser Thr
            420                 425                 430

Gly Leu Phe Thr Ile Asn Ser Ala Leu Val Ile Asp Leu Tyr Pro Gly
        435                 440                 445

Ala Ser Ala Ser Ala Thr Ala Val Asn Asn Leu Met Arg Cys Leu Leu
    450                 455                 460

Gly Ala Gly Gly Val Ala Val Val Gln Pro Ile Leu Asp Ala Leu Asn
465                 470                 475                 480

Pro Asp Tyr Thr Phe Leu Leu Leu Ala Gly Ile Thr Leu Val Met Thr
                485                 490                 495

Pro Leu Leu Tyr Val Glu Asp Arg Trp Gly Pro Gly Trp Arg His Ala
            500                 505                 510

Arg Glu Arg Arg Leu Lys Ala Lys Ala Asn Gly Asn
        515                 520

<210>  20
<211>  524
<212>  PRT
<213>  Aspergillus niger

<400>  20

Met Ser Ser Thr Thr Ser Ser Ser Arg Ser Asp Leu Glu Lys Val Pro
1               5                   10                  15

Val Pro Gln Val Ile Pro Arg Asp Ser Asp Ser Asp Lys Gly Ser Leu
            20                  25                  30

Ser Pro Glu Pro Ser Thr Leu Glu Ala Gln Ser Ser Glu Lys Pro Pro
        35                  40                  45

His His Ile Phe Thr Arg Ser Arg Lys Leu Gln Met Val Cys Ile Val

50                  55                  60

Ser Leu Ala Ala Ile Phe Ser Pro Leu Ser Ser Asn Ile Tyr Phe Pro
65                  70                  75                  80

Ala Leu Asp Asp Val Ser Lys Ser Leu Asn Ile Ser Met Ser Leu Ala
                85                  90                  95

Thr Leu Thr Ile Thr Val Tyr Met Ile Val Gln Gly Leu Ala Pro Ser
            100                 105                 110

Phe Trp Gly Ser Met Ser Asp Ala Thr Gly Arg Arg Pro Val Phe Ile
        115                 120                 125

Gly Thr Phe Ile Val Tyr Leu Val Ala Asn Ile Ala Leu Ala Glu Ser
    130                 135                 140

Lys Asn Tyr Gly Glu Leu Met Ala Phe Arg Ala Leu Gln Ala Ala Gly
145                 150                 155                 160

Ser Ala Ala Thr Ile Ser Ile Gly Ala Gly Val Ile Gly Asp Ile Thr
                165                 170                 175

Asn Ser Glu Glu Arg Gly Ser Leu Val Gly Ile Phe Gly Gly Val Arg
            180                 185                 190

Met Leu Gly Gln Gly Ile Gly Pro Val Phe Gly Gly Ile Phe Thr Gln
        195                 200                 205

Tyr Leu Gly Tyr Arg Ser Ile Phe Trp Phe Leu Thr Ile Ala Gly Gly
    210                 215                 220

Val Ser Leu Leu Ser Ile Leu Val Leu Leu Pro Glu Thr Leu Arg Pro
225                 230                 235                 240

Ile Ala Gly Asn Gly Thr Val Lys Leu Asn Gly Ile His Lys Pro Phe
                245                 250                 255

Ile Tyr Thr Ile Thr Gly Gln Thr Gly Val Val Glu Gly Ala Gln Pro
            260                 265                 270

Glu Ala Lys Lys Thr Lys Thr Ser Trp Lys Ser Val Phe Ala Pro Leu
        275                 280                 285

Thr Phe Leu Val Glu Lys Asp Val Phe Ile Thr Leu Phe Phe Gly Ser
    290                 295                 300

Ile Val Tyr Thr Val Trp Ser Met Val Thr Ser Ser Thr Thr Asp Leu
305                 310                 315                 320

Phe Ser Glu Val Tyr Gly Leu Ser Ser Leu Asp Ile Gly Leu Thr Phe
                325                 330                 335

Leu Gly Asn Gly Phe Gly Cys Met Ser Gly Ser Tyr Leu Val Gly Tyr
            340                 345                 350

Leu Met Asp Tyr Asn His Arg Leu Thr Glu Arg Glu Tyr Cys Glu Lys
        355                 360                 365

His Gly Tyr Pro Ala Gly Thr Arg Val Asn Leu Lys Ser His Pro Asp
    370                 375                 380

Phe Pro Ile Glu Val Ala Arg Met Arg Asn Thr Trp Trp Val Ile Ala
385                 390                 395                 400

Ile Phe Ile Val Thr Val Ala Leu Tyr Gly Val Ser Leu Arg Thr His
                405                 410                 415

Leu Ala Val Pro Ile Ile Leu Gln Tyr Phe Ile Ala Phe Cys Ser Thr
            420                 425                 430

Gly Leu Phe Thr Ile Asn Ser Ala Leu Val Ile Asp Leu Tyr Pro Gly
        435                 440                 445

Ala Ser Ala Ser Ala Thr Ala Val Asn Asn Leu Met Arg Cys Leu Leu
    450                 455                 460

Gly Ala Gly Gly Val Ala Ile Val Gln Pro Ile Leu Asp Ala Leu Lys
465                 470                 475                 480

Pro Asp Tyr Thr Phe Leu Leu Leu Ala Gly Ile Thr Leu Val Met Thr
                485                 490                 495

Pro Leu Leu Tyr Val Glu Asp Arg Trp Gly Pro Gly Trp Arg His Ala
            500                 505                 510

Arg Glu Arg Arg Leu Lys Ala Lys Ala Asn Gly Asn
        515                 520

<210>  21
<211>  23
<212>  DNA
<213>  Artificial Sequence

<220>
<223>  CRISPR targeting sequences

<400> 21
cgtgtgaaga tatgatgcgg tgg 23